# EUROPEAN PATENT APPLICATION

(11) **EP 1 702 886 A1**
(43) Date of publication of application: **20.09.2006**
(21) Application number: 04773218.5
(22) Date of filing: 10.09.2004
(51) Int. Cl.: C01B 33/12

(54) **POROUS SILICA HAVING SUBSTANCE CARRIED THEREON**

(30) Priority: 11.09.2003 JP 2003320436; 05.02.2004 JP 2004029981; 05.02.2004 JP 2004029984; 05.02.2004 JP 2004029986; 05.02.2004 JP 2004029990
(71) Applicant: Taiyo Kagaku Co., Ltd., Yokkaichi-shi Mie 510-0825 (JP)
(72) Inventor: KITAHATA, Kouichi, c/o Taiyo Kagaku Co., Ltd., Yokkaichi-shi, Mie 510-0825 (JP); TERAMOTO, Kanae, c/o Taiyo Kagaku Co., Ltd., Yokkaichi-shi, Mie 510-0825 (JP); YANAGI, Masaaki, c/o Taiyo Kagaku Co., Ltd., Yokkaichi-shi, Mie 510-0825 (JP); NANBU, Hironobu, c/o Taiyo Kagaku Co., Ltd., Yokkaichi-shi, Mie 510-0825 (JP); YAMAZAKI, Yoshiki, c/o Taiyo Kagaku Co., Ltd., Yokkaichi-shi, Mie 510-0825 (JP); HORII, Mitsumasa, Aichi-gun, Aichi 480-1131 (JP); FUKUSHIMA, Yoshiaki, Aichi-gun, Aichi 480-1113 (JP)
(74) Representative: HOFFMANN EITLE
(86) International application number: PCT/JP2004/013574
(87) International publication number: WO 2005/026048

(57) **Abstract**

The present invention relates to a substance-supporting porous silica, wherein a porous silica supports a substance selected from the group consisting of menthols, volatile substances, thermal substances, plant polyphenols and organic colorants.

## Description

### TECHNICAL FIELD

The present invention relates to a substance-supporting porous silica and a composition containing the porous silica.

### BACKGROUND ART

Menthol has refreshing feel or cool feel, so that the menthol has been used for various foodstuff, pharmaceuticals, cosmetics, luxury items, toiletry articles and the like. However, since the menthol has sublimation property and volatility, the menthol may undergo sublimation due to a temperature change or the like during storage of the manufactured articles, thereby resulting in reduction of its content. Also, after sublimation, the menthol may recrystallize in a container. Since the formed crystals are acicular crystals, the value of the manufactured article is dropped if the crystals appear to be in a cobweb form.

Especially, in a food such as chewing gum, the manufacture of a food which strongly appeals refreshing feel or cool feel of menthol and has favorably remaining feels such as the refreshing feel or cool feel increases its commercial value in light of meeting the demands of consumers.

The chewing gum exhibits its flavor by physical compression due to mastication and dissolution of sugar with saliva. However, as the gum is continued chewing, its taste becomes gradually weaker, so that not only flavor but also sweetness, sourness, refreshing feel or the like is undesirably lowered after several minutes of chewing. Therefore, in order to manufacture chewing gum which can appeal its refreshing feel or cool feel of menthol at the beginning of chewing, a means of increasing the amount of menthol has been usually employed.

However, even if the amount of menthol is increased, there are some disadvantages that the content of the menthol is reduced during storage or the menthol is undesirably adsorbed in the texture of chewing gum, so that its volatility in the mouth is worsened, and that almost an entire amount is eluted to volatilize at the beginning of chewing, thereby being deficient in sustainability.

In view of the above, as a process for suppressing sublimation and volatilization of flavor components such as menthol, a process comprising mixing a flavor component with a fat or oil or a solvent, having a boiling point higher than a sublimation temperature and/or volatilization temperature of the flavor component (for example, see Japanese Patent Laid-Open No. Hei 11-50084) has been known. In addition, in order to sustain refreshing feel or cool feel, there have been proposed cosmetics in which a refreshing agent such as menthol is included with a cyclodextrin derivative (for example, see Japanese Patent Laid-Open No. Hei 6-329528), a process comprising blending menthol with a dioxolan-2-acetic acid derivative (for example, see Japanese Patent Laid-Open No. Hei 7-228887), a process comprising blending menthol with a tea tree refined oil (for example, see Japanese Patent Laid-Open No. Hei 9-263786), a process comprising blending a cool feel-giving substance such as menthol with vanillyl butyl ether (for example, see Japanese Patent Laid-Open No. 2000-44924), a process comprising blending menthol with an N-substituted-p-menthanecarboxamide (for example, see Japanese Patent Laid-Open No. Hei 3-53849) and the like.

However, while the objective of sustaining a refreshing feel or cool feel for a long period of time is improved to some extent, the results are not necessarily sufficiently satisfactory.

As refrigerants, a cooling gel sheet comprising a substrate made of a nonwoven fabric or the like, and a water retention layer made of a water-containing polymer gel, which is used in plastering on an affected part upon, for example, abrupt onset of fever in infants; an icing agent (gel, ointment) for suppressing inflammation of muscles after having played sports; and the like are commercially available. Also, there is a refrigerant in which is a water-absorbed sheet is wound around a container such as can, bin or PET bottle for cooling a beverage in the container. The cooling gel sheet has the features that the cooling gel sheet can be plastered on a part to be cooled such as forehead without being peeled off even when the infants move, so that this cooling gel sheet can be allowed to stand as it is during a period of time capable of cooling because the water-containing polymer gel has adhesion.

The cooling gel sheet as described above usually comprises a substrate made of a nonwoven fabric or the like and a polymer gel layer having a water content of about 70 to about 90% by weight, and heat is taken away from a plastered member in the form of a latent heat of vaporization when water contained in this polymer gel is evaporated, thereby cooling the plastered member. Some of the sheets contain a volatile component such as menthol other than water to enhance the cooling effect. Therefore, the larger the amounts of water and the volatile component contained in the polymer gel layer, the longer the period of time capable of cooling and the more enhanced the cooling effect, so that the cooling gel sheet is especially effective for the purpose of quick cooling.

However, if the water content of the polymer gel is increased, the water content would undesirably exceed a tolerable water content of the gel, thereby worsening stability of the polymer gel layer, whereby it is made difficult to keep self-shape retaining ability and adhesion of the gel. Therefore, there arises a trouble that the polymer gel layer adheres to a side of skin or the like. On the other hand, even if the content of the volatile component is increased, the volatile component undesirably vaporizes during storage, so that it has been difficult to allow the volatile component to remain in a high content until actual use.

As improving means thereof, there have been proposed a process in which a polyvinyl alcohol is used for a gelling agent (for example, see Japanese Patent Laid-Open No. Hei 6-7395), a process in which a compound capable of endothermically dissolving in water is used (for example, see Japanese Patent Laid-Open No. 2000-107219), a process comprising arranging the water-containing gel layer to a side not plastered on a plastered member (for example, see Japanese Patent Laid-Open No. 2000-189451) and the like.

These conventional proposals attain their objectives to some extent, but the results were not necessarily satisfactory.

As thermal agents, a thermal gel sheet comprising a substrate made of a nonwoven fabric or the like, and a water retention layer having a water-containing polymer gel, which is used for plastering on an affected part upon experiencing shoulder stiffness, arthralgia, lower backache, myalgia, muscle fatigue, bruise, sprain, frostbite or the like; a blood circulation accelerator (gel, cream or ointment) in cases of poor circulation in limbs or shoulder; and the like are commercially available. Gloves, socks or the like for poor circulation have been also commercially available.

In the thermal agent as described above, commonly, a blood circulation accelerator such as capsicum extract, ginger extract, ginger oil, capsaicin, vanillylamide nonylate, a vanillyl alcohol alkyl ether (alkyl group of which has 3 to 6 carbon atoms), camphor, or nicotinic acid amide has been used. Since the thermal agent gives a very strong stimulus, a composition for moderating the stimulus, containing a fatty acid ester or the like (for example, see Japanese Patent Laid-Open No. 2001-19608) has been proposed.

However, these conventional proposals attain their objectives to some extent, but the results were not necessarily satisfactory.

As the odor components generated by smoking or the like, about twenty-odd kinds have been known of those that are identified. The main components thereof are three kinds: acetic acid, ammonia and acetaldehyde. Among them, acetic acid and ammonia can be relatively easily removed by neutralizing with an acidic or basic substance, respectively; however, it is said to be difficult to remove acetaldehyde which is a neutral substance.

In addition, according to the improvement in airtightness of rooms accompanying changes in building structures, there is an increasing concern for a so-called sick house syndrome, that an easily volatile substance (VOC) generated from an adhesive used for wallpaper, a new building material or the like, especially formaldehyde, affects on human bodies, and de-formalin formation for buildings to cope with this concern is progressing. However, its progress is very slow, and measures for existing buildings constructed without subjecting to de-formalin formation treatment yet remain almost untouched at present. Moreover, there is also a disadvantage that since the formaldehyde is a neutral substance in the same manner as the above-mentioned acetaldehyde, its removal is not easy.

In addition, similarly, according to the improvement in airtightness of rooms, there is an increasing concern that influenza or the like can be more likely to be easily infected than before, so that especially moisture regulation, antibacterial treatment and deodorization in the room environment are becoming important issues in future.

Furthermore, with the increase in inclination of cleanliness of these days, many manufactured articles expressly having antibacterial ability are likely to be commercialized in many fields. On the contrary, however, the safety of the antibacterial agent itself which is added to the manufactured article is said to be questionable, so that safe and a so-called human-friendly antibacterial agent is in demand.

The plant polyphenol represented by, for example, an extract component of tea or an extract component of fruit such as persimmon or apple, which is usually a mixture of a plurality of polyphenols such as tannins, catechins and flavonoids, is a natural occurring component that is known to have effects of having excellent safety and suppressing propagation of bacteria or viruses, in other words, having antibacterial effects, and moreover having strong deodorization effects against odor components such as ammonia, an amine, and an aldehyde.

In view of the above, use of a plant polyphenol as an antibacterial deodorant by adhering the plant polyphenol on the surface of fibers constituting a filter for air cleaners (for example, see Japanese Patent Laid-Open Nos. Hei 10-315 and Hei 9-141021) has been studied.

However, although the plant polyphenol exhibits excellent antibacterial and deodorizing effects in the presence of water, there are some risks that the antibacterial and deodorizing effects are not satisfactorily exhibited in the above constitutions depending on the operable circumstances (especially humidity conditions upon use) because the plant polyphenol does not well function in a dried state.

In addition, when the plant polyphenol is used in a literally exposed state as mentioned above, the plant polyphenol is gradually deactivated by undergoing oxidative degradation, hydrolysis or the like, so that there is a disadvantage in the sustainability of the effects. As a means of avoiding this disadvantage, an antibacterial deodorant comprising a plant polyphenol being held by an activated carbon in the state of an aqueous solution (for example, see Japanese Patent Laid-Open No. Hei 8-291013) has been studied. However, there is a disadvantage that the activated carbon, especially in a dry environment, cannot retain water over a long period of time and undesirably releases water in a short period of time, at which point the antibacterial and deodorizing effects are markedly lowered. Therefore, in fact, there is a disadvantage that the sustainability of the effects is not much improved.

Also, a process comprising retaining a plant polyphenol to amorphous calcium phosphate or a hygroscopic substance (for example, see Japanese Patent Laid-Open Nos. 2000-135277 and 2000-327512) has been studied. However, this process has a disadvantage of over-absorption of water under high-humidity conditions, and does not satisfy all of hygroscopicity regulatory function, deodorization function, and adsorption function of chemical substances, so that further improvements therefor have been desired.

Colorants are roughly classified into two kinds of dyes and pigments, depending upon whether or not the particles insoluble in water, an alcohol or an oil are present in the colorant.

Conventionally, the dye which does not contain the particles insoluble in water, an alcohol or an oil give a high color development but has a disadvantage that the dye has worsened water resistance and light fastness.

On the other hand, the pigment which contains the particles insoluble in water, an alcohol or an oil has excellent water resistance and light fastness but has a disadvantage that the pigment has worsened color development.

Especially in printouts using inkjet inks, the obtainment of the printouts with inks which together have color development of the dye and water resistance and light fastness of the pigment increases the commercial values from the viewpoint of meeting the demands of the consumers.

As the process of supplementing the shortcomings as described above, a process of improving water resistance and light fastness of an ink, comprising working on a side of a recording sheet in such a manner that a recording sheet contains a mesoporous silica having an average pore size of more than 10 nm and less than 35 nm (for example, see Japanese Patent Laid-Open No. 2001-179086) has been known.

The effects of improving water resistance and light fastness of the printouts printed on the recording sheet as described above are, however, not sufficiently satisfied.

On the other hand, as a process comprising working on a side of a colorant, for example, a process for preparing a pigment composition obtained from a layered clay mineral represented by such as montmorillonite and mica, and a water-soluble dye, and cosmetics (for example, see Japanese Patent Laid-Open Nos. Sho 53-113036, Sho 61-111367 and Sho 63-90573) have been known.

The colored pigment obtained by the process described above, however, cannot be said to have sufficient light fastness and has a disadvantage that a secondary aggregation by the pigments themselves is likely to take place. Therefore, the objective of sustaining of color development, water resistance and light fastness of the colorant for a long period of time is improved to a certain extent, the effects are not necessarily sufficiently satisfactory.

### DISCLOSURE OF THE INVENTION

### PROBLEMS TO BE SOLVED BY THE INVENTION

An object of the invention is to provide a substance-supporting porous silica which has excellent adsorbability of the substance and mild desorbability or controlled-release property by a physical or chemical stimulus from external, and a composition comprising the porous silica.

### MEANS TO SOLVE THE PROBLEMS

Specifically, the present invention relates to:
[1] a substance-supporting porous silica, wherein a porous silica supports a substance selected from the group consisting of menthols, volatile substances, thermal substances, plant polyphenols and organic colorants;
[2] the substance-supporting porous silica according to the above [1], further comprising an emulsifying agent;
[3] the substance-supporting porous silica according to the above [1] or [2], wherein the porous silica has a pore having an average pore size of from 0.8 to 20 nm;
[4] the substance-supporting porous silica according to any one of the above [1] to [3], wherein the porous silica has an average particle size of from 50 nm to 100 µm;
[5] the substance-supporting porous silica according to any one of the above [1] to [4], wherein the pore of the porous silica forms a hexagonal structure;
[6] a composition comprising the substance-supporting porous silica as defined in any one of the above [1] to [5];
[7] a coolant comprising a porous silica;
[8] a menthol-supporting porous silica, wherein a porous silica having a pore having an average pore size of from 0.8 to 20 nm supports a menthol;
[9] the porous silica according to the above [8], further comprising an emulsifying agent;
[10] a menthol-containing composition comprising the porous silica as defined in the above [8] or [9];
[11] the menthol-containing composition according to the above [10], wherein the menthol-containing composition is at least one member selected from the group consisting of foodstuff, pharmaceuticals, cosmetics, luxury items and toiletry articles;
[12] the composition according to the above [11], wherein the foodstuff are chewing gum;
[13] a composition for a refrigerant, characterized in that the composition comprises a porous silica;
[14] a volatile substance-supporting porous silica, wherein a porous silica having a pore having an average pore size of from 0.8 to 20 nm supports a volatile substance;
[15] the porous silica according to the above [14], wherein the porous silica has an average particle size of from 50 nm to 100 µm;
[16] a composition comprising the porous silica as defined in the above [14] or [15];
[17] a thermal substance-supporting porous silica, wherein a porous silica having a pore having an average pore size of from 0.8 to 20 nm supports a thermal substance;
[18] the porous silica according to the above [17], wherein the porous silica has an average particle size of from 50 nm to 100 µm;
[19] the porous silica according to the above [17] or [18], wherein the thermal substance is a capsicum extract;
[20] a composition comprising the porous silica as defined in any one of the above [17] to [19];
[21] a plant phenol-supporting porous silica, wherein a porous silica having a pore having an average pore size of from 0.8 to 20 nm, the pore forming a hexagonal structure, supports a plant polyphenol;
[22] a composition comprising the porous silica as defined in the above [21];
[23] an organic colorant-supporting porous silica, wherein a porous silica having a pore having an average pore size of from 0.8 to 20 nm, the pore forming a hexagonal structure, and having an average particle size of from 50 nm to 10 µm, supports an organic colorant;
[24] the porous silica according to the above [23], further comprising an emulsifying agent;
[25] the porous silica according to the above [23] or [24], wherein the emulsifying agent is a polyglycerol fatty acid ester, in which 70% or more of the polyglycerol constituting the polyglycerol fatty acid ester has a degree of polymerization of 3 or more;
[26] a composition comprising the porous silica as defined in any one of the above [23] to [25]; and
[27] the composition according to the above [26], wherein the composition is an ink, foodstuff, or cosmetics.

### EFFECTS OF THE INVENTION

According to the present invention, a substance-supporting porous silica which has excellent adsorbability of the substance and mild desorbability or controlled-release property by a physical or chemical stimulus from external, and a composition comprising the porous silica can be provided. Since the substance-supporting porous silica of the present invention has excellent adsorbability of the substance and mild desorbability or strong controlled-release property by a physical or chemical stimulus from external, the release of the substance can be controlled.

### BEST MODE FOR CARRYING OUT THE INVENTION

The substance-supporting porous silica of the present invention comprises a porous silica supporting a specified substance. The substance which can be supported to the porous silica of the present invention is a substance selected from the group consisting of menthols, volatile substances, thermal substances, plant polyphenols and organic colorants.

The embodiments of the porous silica supporting each of the substances will be explained hereinbelow.

### (First Embodiment)

First, a first embodiment in which a substance to be supported is menthol will be explained. In this embodiment, a menthol-supporting porous silica having enhanced refreshing feel and cool feel, and excellent sustainability of the feels, and a composition containing the porous silica are provided.

In this embodiment, the menthol is not particularly limited as long as the menthol is a menthol and/or a menthol-containing refined oil. The menthol includes natural menthol, synthetic menthol, mentha herb oil, peppermint oil, spearmint oil, and the like. These can be used alone or in admixture of two or more kinds.

The porous silica in this embodiment has an average pore size of from 0.8 to 20 nm, preferably from 0.8 to 10 nm, more preferably from 1 to 10 nm, and even more preferably from 2 to 5 nm, from the viewpoint of the adsorbed amount of the menthol onto the porous silica and the sustainability of the menthol to be adsorbed on the porous silica.

The pore of the porous silica is not particularly limited, and it is preferable that the pore is in the form of a hexagonal structure. The shape of the pore can be confirmed by an X-ray diffraction or the like.

The porous silica has a pore volume of preferably from 0.1 to 3.0 cm³/g, and more preferably from 0.2 to 2.0 cm³/g, from the viewpoint of the adsorbed amount of menthol onto the porous silica.

The porous silica has a specific surface area of preferably from 400 to 1500 m²/g, more preferably from 600 to 1500 m²/g, and more preferably from 600 to 1200 m²/g, from the viewpoint of the adsorbed amount of menthol onto the porous silica.

The average pore size, pore volume and specific surface area of the porous silica in this embodiment are obtained from a nitrogen adsorption isotherm determined by a known BET method.

The porous silica in this embodiment has an average particle size of preferably from 50 nm to 100 µm, more preferably from 50 nm to 10 µm, even more preferably from 50 nm to 5 µm, still even more preferably from 50 to 500 nm, and still even more preferably from 50 to 300 nm. The average particle size can be determined by a laser method or a dynamic light scattering method.

The method for preparing a porous silica in this embodiment is not particularly limited, and includes, for example, a process comprising mixing an inorganic raw material with an organic raw material to react the mixture, thereby forming a complex of the organic substance and the inorganic substance in which the organic substance is used as a template, and the backbone of the inorganic substance is formed in the surrounding of the organic substance, and removing the organic substance from the resulting complex.

The inorganic raw material is not particularly limited as long as the substance contains silicon. The silicon-containing substance includes, for example, substances containing a silicate such as a layered silicate or a non-layered silicate, and substances containing silicon other than the silicate. The layered silicate includes kanemite (NaHSi₂O₅·3H₂O), sodium disilicate crystals (Na₂Si₂O₅), makatite (NaHSi₄O₉·5H₂O), ilerite (NaHSi₈O₁₇·XH₂O), magadiite (Na₂HSi₁₄O₂₉·XH₂O), kenyaite (Na₂HSi₂₀O₄₁·XH₂O) and the like. The non-layered silicate includes water glass (sodium silicate), glass, amorphous sodium silicate, and silicon alkoxides such as tetraethoxysilane (TEOS), tetramethylammonium (TMA) silicate and tetraethyl orthosilicate, and the like. In addition, the substance containing silicon other than the silicate includes silica, oxide of silica, silica-metal composite oxides and the like. These inorganic raw materials may be used alone or in admixture of two or more kinds.

The organic raw material includes cationic, anionic, amphoteric, and nonionic surfactants, high-molecular polymers, and the like. The organic raw material can be used alone or in admixture of two or more kinds.

The cationic surfactant includes primary amine salts, secondary amine salts, tertiary amine salts, quaternary amine salts and the like. Among them, the quaternary amine salts are preferable. Since an amine salt has poor dispersibility in an alkaline region, the amine salt is used only where the preparation condition is acidic. However, the quaternary amine salt can be used in both cases where the preparation condition is alkaline and acidic.

As the quaternary amine salt, an alkyl(8 to 22 carbon atoms)trimethylammonium salt such as octyltrimethylammonium chloride, octyltrimethylammonium bromide, octyltrimethylammonium hydroxide, decyltrimethylammonium chloride, decyltrimethylammonium bromide, decyltrimethylammonium hydroxide, dodecyltrimethylammonium chloride, dodecyltrimethylammonium bromide, dodecyltrimethylammonium hydroxide, hexadecyltrimethylammonium chloride, hexadecyltrimethylammonium bromide, hexadecyltrimethylammonium hydroxide, octadecyltrimethylammonium chloride, octadecyltrimethylammonium bromide, octadecyltrimethylammonium hydroxide, behenyltrimethylammonium chloride, behenyltrimethylammonium bromide, behenyltrimethylammonium hydroxide, tetradecyltrimethylammonium chloride, tetradecyltrimethylammonium bromide, tetradecyltrimethylammonium hydroxide, benzyltrimethylammonium chloride, benzyltrimethylammonium bromide or benzyltrimethylammonium hydroxide, is preferable.

The anionic surfactant includes carboxylates, sulfuric ester salts, sulfonates, phosphoric ester salts and the like. Among them, soaps, higher alcohol sulfuric ester salts, higher alkyl ether sulfuric ester salts, sulfated oils, sulfated fatty acid esters, sulfated olefins, alkylbenzenesulfonates, alkylnaphthalenesulfonates, paraffinic sulfonates and higher alcohol phosphoric ester salts are preferable. These anionic surfactants can be used alone or in admixture of two or more kinds.

As the amphoteric surfactant, sodium laurylaminopropionate, stearyldimethylbetaine, lauryldihydroxyethylbetaine and the like are preferable. These amphoteric surfactants can be used alone or in admixture of two or more kinds.

As the nonionic surfactant, those in the form of ethers such as polyoxyethylene alkyl ethers, polyoxyethylene secondary alcohol ethers, polyoxyethylene alkyl phenyl ethers, polyoxyethylene sterol ethers, polyoxyethylene lanolin acid derivatives, polyoxyethylene polyoxypropylene alkyl ethers, polypropylene glycol and polyethylene glycol; and those in the form of containing nitrogen such as polyoxyethylene alkylamine are preferable. These nonionic surfactants can be used alone or in admixture of two or more kinds.

When a substance containing silicon other than the layered silicate, for example, silicon oxides such as silica (SiO₂), is used as the inorganic raw material, the pores can be formed by firstly forming a layered silicate such as kanemite, inserting a template made of an organic substance between the layers, connecting the layers where the template is not present with silicate molecules, and thereafter removing the template made of the organic substance therefrom. In addition, when a non-layered silicate such as water glass is used, pores can be formed by gathering silicate monomers in the surrounding of the template, polymerizing the monomers to form silica, and thereafter removing the template therefrom.

On the other hand, when a surfactant is used as an organic material and pores are formed by using the surfactant as a template, micelles thereof can be utilized as the template. When the alkyl chain length of the surfactant is controlled, the diameter of the template changes, whereby the size of the pores formed can also be controlled. Furthermore, a relatively hydrophobic molecule such as trimethylbenzene or tripropylbenzene is added together with the surfactant, so that the micelles expand, whereby pores having even larger sizes can be formed. Pores having an optimal size can be formed for the menthol to be supported by utilizing these methods.

When mixing the inorganic raw material with the organic raw material, a proper solvent may be used. The solvent is not particularly limited, and includes, for example, water, alcohols and the like.

The method of removing the organic substance from the complex of the organic and inorganic substances includes a method comprising collecting the complex by filtration, washing the complex with water or the like, drying the complex, and thereafter baking the dried complex at preferably from 400° to 800°C, and more preferably from 400° to 600°C; and a method comprising extracting the organic substance with an organic solvent.

The porous silica in this embodiment may bound and support to a compound having an ethoxy (or methoxy) group capable of giving a silanol group by hydrolysis on one end, and an organic functional group such as amino group and glycidyl group on the other end, as occasion demands. As a specific example, it is preferable that an amino group-containing silicon compound is bound to and supported by the porous silica. The amino group-containing silicon compound includes compounds having one or more amino groups and one bindable functional group to be subjected to binding with a hydroxyl group on the surface of the porous silica, for example, (3-aminopropyl)methylethoxysilane; compounds having two or more amino groups, for example, bis(3-aminopropyl)methylethoxysilane and tris(3-aminopropyl)ethoxysilane; and the like. The method in which the amino group-containing silicon compound is bound and supported to the porous silica is not particularly limited. For example, the compound is mixed with the porous silica while dispersing in water or the like to support the compound, and the mixture may be further dried as occasion demands.

In this embodiment, the method of supporting menthol on the porous silica is not particularly limited, and includes, for example, a method comprising mixing the porous silica with a solution prepared by dissolving menthol in a proper solvent, and further removing the solvent to dryness as occasion demands; a method comprising placing the porous silica and menthol in a tightly sealed reduced pressure vessel, and allowing the menthol to sublime and at the same time adsorbing the menthol onto the porous silica; and the like. When the menthol is supported to the porous silica, it is preferable that the preparation is carried out at 40°C or more in order to enhance the strength of adsorbing the menthol without adsorbing moisture in the air as much as possible. The term "mixing" as used herein refers to an appropriate selection of a commonly used mixing machine such as a mixer or a kneader, and homogeneous mixing with the mixing machine. The mixing conditions are appropriately set depending upon the compositional ratio, the amount of the mixture, or the like.

The content of the menthol is not particularly limited. The content is preferably 0.01 parts by weight or more, based on 100 parts by weight of the porous silica (on a solid basis), from the viewpoint of efficiently exhibiting a refreshing feel or cool feel of the menthol-supporting porous silica of this embodiment without spoiling taste. The content of the menthol is preferably 50 parts by weight or less, based on 100 parts by weight of the porous silica (on a solid basis), from the viewpoint of sustainability of adsorption of the menthol. Therefore, from the viewpoint mentioned above, the content of the menthol is preferably from 0.01 to 100 parts by weight, more preferably from 1 to 80 parts by weight, and even more preferably from 20 to 80 parts by weight, based on 100 parts by weight of the porous silica (on a solid basis).

Moreover, it is preferable that an emulsifying agent is contained in the menthol-supporting porous silica of this embodiment, from the viewpoint of enhancing strength and sustainability of a refreshing feel or cool feel and improving dispersibility in water.

As the emulsifying agent, a generally known emulsifying agent can be used without particular limitation. The emulsifying agent includes, for example, glycerol fatty acid esters, polyglycerol fatty acid esters, sucrose fatty acid esters, sorbitan fatty acid esters, propylene glycol fatty acid esters, lecithin, enzymatically decomposed lecithin and the like. These emulsifying agents can be used alone or in admixture of two or more kinds. Among them, the polyglycerol fatty acid esters are preferable.

The emulsifying agent has an HLB value of preferably 10 or more, and more preferably from 15 to 20, from the viewpoint of improving dispersibility. When two or more kinds of the emulsifying agents are used, the average HLB value thereof is preferably 10 or more, and more preferably from 15 to 20.

When the porous silica of this embodiment further contains an emulsifying agent, it is preferable that the emulsifying agent is selectively adsorbed onto the external of the menthol-supporting porous silica without penetrating into the pores of the porous silica as much as possible, from the viewpoint of sustainability of a refreshing feel or cool feel. The emulsifying agent is preferably a polyglycerol fatty acid ester obtained by esterification of a polyglycerol having an average degree of polymerization of 3 or more and a fatty acid having 12 or more carbon atoms, more preferably a polyglycerol fatty acid ester obtained by esterification of a polyglycerol having an average degree of polymerization of 3 or more and a fatty acid having 16 or more carbon atoms, and even more preferably a polyglycerol fatty acid ester obtained by esterification of a polyglycerol having an average degree of polymerization of 3 or more and a fatty acid having 18 or more carbon atoms, from the viewpoint of selectively adsorbing the menthol to the external of the porous silica. Among the above-mentioned polyglycerol fatty acid esters, a polyglycerol fatty acid ester in which 2 to 10 fatty acid molecules are esterified is even more preferable. These emulsifying agents may be used alone or in combination of two or more kinds.

The content of the emulsifying agent is not particularly limited. For example, the content is preferably from 0.01 to 50 parts by weight, and more preferably from 0.1 to 30 parts by weight, based on 100 parts by weight (on a solid basis) of the composition in which the menthol is supported to the porous silica. From the viewpoint of sustainability of a refreshing feel or cool feel, the content is even more preferably from 0.1 to 10 parts by weight, and still even more preferably from 1 to 10 parts by weight, based on 100 parts by weight (on a solid basis) of the composition.

The timing of the addition of the emulsifying agent when preparing the menthol-supporting porous silica of this embodiment is not particularly limited. The emulsifying agent may be added together with the menthol when the menthol is supported to the porous silica, or the emulsifying agent may also be added after the menthol is supported thereto. It is preferable that the emulsifying agent is added after the menthol is supported thereto, from the viewpoint of more remarkably obtaining effects by adding the emulsifying agent.

The method of adding the emulsifying agent is also not particularly limited. When the emulsifying agent is in a liquid state, the emulsifying agent can be blended with the menthol-supporting porous silica simply by kneading. Therefore, from the viewpoint of more homogeneously adding the emulsifying agent to the menthol-supporting porous silica, preferable are a method comprising adding a dispersion or solution, prepared by dispersing or dissolving an emulsifying agent in a solvent such as ethanol or water, and removing the solvent therefrom; and a method comprising adding a dispersion or solution, prepared by dispersing or dissolving an emulsifying agent in a solvent such as ethanol or water.

Further, the menthol-supporting porous silica of this embodiment may contain, besides the menthol, an additive such as an unsaturated fatty acid, a carotenoid, a vitamin, a pigment, a spice, or a derivative thereof, or a composition containing the additive, and other functional substances.

The unsaturated fatty acid includes docosahexaenoic acid, eicosapentaenoic acid, α-linolenic acid, γ-linolenic acid, conjugated linoleic acid, arachidonic acid and the like.

The carotenoid includes β-carotene, α-carotene, γ-carotene, lutein, lycopene, astaxanthin, canthaxanthin and the like.

The vitamin includes vitamin A, vitamin D, vitamin E, vitamin K, tocotrienol and the like.

The pigment include anthocyanin pigments such as hibiscus pigment, red cabbage pigment, purple sweet potato pigment and blueberry pigment; flavonoid pigments such as safflower pigment; carotenoid pigments such as Dunaliella pigment, carrot pigment and palm-derived pigments; chlorella pigment; turmeric pigment; naphthoquinone-based pigments; and the like.

The spice includes spices extracted from capsicum, cardamom, mint, pepper, turmeric, cumin, sage, parsley, oregano, saffron, rosemary, thyme and the like.

The other functional substance includes antioxidants such as lecithin, green tea extracts, oolong tea extracts, black tea extracts, ascorbic acid, erythorbic acid, polyphenol compounds, rosemary extracts, t-butylhydroxytoluene, t-butylhydroxyanisole, tocopherol, tocotrienol and ethoxyquin, various minerals, amino acids and the like.

Further, in the menthol-supporting porous silica of this embodiment, there may be properly blended and processed an additive such as a polysaccharide such as alginic acid, β-glucan, yeast cell wall, guar gum or an enzymatically decomposed guar gum; a protein such as zein, gelatin or casein; a carbohydrate such as dextrin; silica, calcium tertiary phosphate, eggshell calcium, a milk serum mineral, or shellac resin as occasion demands.

The additive may be supported to the porous silica at the same time as the menthol or separately from the menthol.

In addition, when the menthol-supporting porous silica of this embodiment is prepared, the porous silica may be dried using a spray dryer, a rotary dryer, a vacuum dryer, a blast dryer or the like, in order to remove water or a solvent, as occasion demands. Among these dryers, it is preferable to use the vacuum dryer, from the viewpoint of preventing degradation of the menthol.

The form of the menthol-supporting porous silica of this embodiment is not particularly limited, and includes powder, granule, sheet, bulk, film and the like forms.

The menthol-supporting porous silica of this embodiment has the features of showing excellent adsorbability of the menthol and even milder desorbability by a physical or chemical stimulus from external. Therefore, the menthol-supporting porous silica of this embodiment can sustain a refreshing feel or cool feel of the menthol, so that the menthol-supporting porous silica can be used for various manufactured articles.

Accordingly, in one embodiment of this embodiment, a menthol-containing composition comprising the menthol-supporting porous silica of this embodiment is further provided. The menthol-containing composition of this embodiment is preferably at least one member selected from the group consisting of foodstuff, pharmaceuticals, cosmetics, luxury items and toiletry articles.

The foodstuff include snacks and candies such as chewing gum, candies, tablet sweets, gummi candies, chocolates, biscuits and snacks; frozen desserts such as ice creams, sherbets and ice candies; powdered beverages, refreshing drinks, carbonated beverages, luxury beverages and the like. Among them, the effects of this embodiment are more remarkably exhibited by containing the menthol-supporting porous silica of this embodiment in the chewing gum for which the demands for the sustainability of a refreshing feel or cool feel are the strongest.

The pharmaceutical includes medicaments and quasi drugs, such as adhesive preparations, cataplasms, plasters, ointments, emplastrum, suppositories, cream agents, liniments, lotions, aerosols, spirits, healthcare drinks, trochisci, chewable tablets, toothpastes, and mouth washing agents.

The cosmetics include make-up powders, lip creams, colognes, antiperspirants, hairdressings and the like.

The luxury items include tobacco, cigars, smoking paraphernalia such as smoking pipe, tobacco substitutes and the like.

The toiletry articles include bath agents, deodorants, fragrances and the like.

The menthol-containing composition of this embodiment can be prepared in the same manner as a conventional method, except that the menthol-supporting porous silica of this embodiment is used; and the timing and the method of the addition of the menthol-supporting porous silica are not limited as long as a composition which exhibits the desired effects of this embodiment is obtained. When a menthol-containing composition is prepared using the menthol-supporting porous silica that does not contain an emulsifying agent, it is preferable that in addition to the porous silica, an emulsifying agent is further added thereto, to prepare the menthol-containing composition in the same manner as a conventional method. For example, in cases of chewing gum, chewing gum are obtained by combining a base material for chewing gum and the menthol-supporting porous silica of this embodiment and other auxiliary materials as occasion demands, kneading the mixture , and molding the kneaded mixture. As the base material for chewing gum, for example, an elastic body, a wax, an inorganic substance or the like is appropriately selected and used. The elastic body includes, for example, natural rubbers, natural chicle, polyisobutylene, vinyl acetate resins, synthetic rubbers, synthetic elastic bodies, natural elastic bodies and the like. The wax includes rice wax, carnauba wax, microcystalline waxes and the like. These may be used alone or in admixture of two or more kinds. For example, there are widely used as a main ingredient of the base material for chewing gum, vinyl acetate resins in the manufacture of bubble-gum, and natural chicle in the manufacture of regular chewing gum.

The content of the menthol-supporting porous silica in the menthol-containing composition of this embodiment is not particularly limited, and can be appropriately selected depending upon the manufactured articles used and the purposes. For example, in the case of chewing gum, the content is preferably from 0.5 to 20 parts by weight, and more preferably from 1 to 5 parts by weight, based on 100 parts by weight of the entire amount of the raw material chewing gum, from the viewpoint of improvement of the refreshing feel or cool feel, influence on the taste, and the like by the menthol-supporting porous silica of this embodiment.

### (Second Embodiment)

Next, a second embodiment in which a substance to be supported is a volatile substance will be explained. In this embodiment, a volatile substance-supporting porous silica having an enhanced cooling effect, and excellent sustainability of the effect, and a composition comprising the porous silica are provided. Since the volatile substance-supporting porous silica of this embodiment has the features of having excellent supporting ability of a volatile substance and increasing a vaporization rate of the content, the porous silica can sustain a refreshing feel or cool feel. In addition, since the volatile substance is previously supported to the porous silica, when the volatile substance-supporting porous silica is applied to various compositions, the pores thereof are inhibited from being clogged, and the volatile substance is vaporized earlier than water, so that the vaporization rate of water can be increased or maintained due to capillary phenomenon.

The volatile substance in this embodiment includes menthol, flavors and spices, refined oils obtained from woods and the like, and these volatile substances can be used alone or in admixture of two or more kinds. In view of the effects, the menthol is preferable. As the menthol, natural menthol, synthetic menthol, and menthol-containing oils such as mentha oil, peppermint oil and spearmint oil can be used alone or in combination of two or more kinds.

The flavor and the spices include, for example, citrus refined oils such as orange oil, lemon oil, grapefruit oil, lime oil, tangerine oil, lavender oil, mandarin oil and bergamot oil; spice oils such as sage, rosemary, perilla, basil, ginger and Japanese horseradish; oleoresins obtained by subjecting these oils to solvent extraction; aromatic vegetable oils such as coffee oil, roast nut oil and sesame oil; natural or synthetic flavoring compounds such as vanillin, maltol, linalool, graniol, citral and limonene; and the like.

The refined oil obtained from woods includes hiba refined oil, hinoki refined oil, cedar refined oil, pine refined oil and the like.

The average pore size, pore structure, pore volume, specific surface area and average particle size of the porous silica in this embodiment are same as those in the first embodiment.

The porous silica of this embodiment can be prepared in the same manner as in the first embodiment. Also, the volatile substance can be supported to the porous silica in the same manner as in the first embodiment.

The content of the volatile substance is not particularly limited, and is preferably from 0.01 to 50 parts by weight, more preferably from 1 to 40 parts by weight, and even more preferably from 20 to 40 parts by weight, based on 100 parts by weight (on a solid basis) of the porous silica, from the viewpoint of releasing sustainability, lowered irritation and reduction in costs.

Further, it is preferable that in the volatile substance-supporting porous silica of this embodiment, an emulsifying agent is contained, from the viewpoint of improving the cooling effect and the sustainability of the effect, and improving dispersibility in water.

The emulsifying agent in this embodiment is the same as that in the first embodiment.

The timing and the method of the addition of the emulsifying agent when preparing the volatile substance-supporting porous silica of this embodiment are the same as those in the first embodiment.

Further, the volatile substance-supporting porous silica of this embodiment may contain, besides the above-mentioned volatile substances, one or more kinds selected from medicaments and functional substances having analgesic effect, anti-inflammatory effect, moisturizing effect, blood-circulation accelerating effect, whitening effect, bactericidal, bacteriostatic and sterilizing effects, antibiotics, antihistamines and the like, such as ethyl aminobenzoate, allantoin, isopropylmethylphenol, indomethacin, ufenamate, camphor, griseofulvin, dipotassium glycyrrhizinate, glycyrrhetinic acid, glycyrrhetinyl stearate, crotamiton, chloramphenicol, chlorhexidine, chlorhexidine hydrochloride, ketoprofen, gentamicin, salicylic acid, ethyleneglycol salicylate, glycol salicylate, diphenhydramine salicylate, methyl salicylate, CSAP, diphenylimidazole, diphenhydramine, diphenhydramine hydrochloride, tannic acid, diphenhydramine tannate, thymol, tetracycline, trehalose, nonylic acid vanillyl amide, hamamelis, sodium hyaluronate, Biosol, piroxicam, felbinac, bufexamac, fluocinolone acetonide, chlorpheniramine maleate, diphenhydramine laurylsulfate, dequalinium chloride, benzalkonium chloride, isothibenzyl hydrochloride, dibucaine hydrochloride, dexamethasone acetate, tocopherol acetate, hydrocortisone acetate, hydrocortisone butyrate, prednisolone valerate, prednisolone valerate acetate, prednisolone acetate, fradiomycin sulfate, urea, *Angelica keiskei* extract, hydrangea tea extract, aloe extract, *Aloe vera* extract, *Ginkgo biloba* extract, F*oeniculum vulgare* fruit extract, oolong tea extract, turmeric extract, scutellaria root extract, *Phellodendri cortex* extract, coptis rhizome extract, *Panax ginseng* root extract, *Hypericum erectum* extract, *Nasturtium officinale* extract, oryzanol, *Anthemis nobilis* flower extract, *Artemisia capillaris* flower extract, *Aloe arborescens* extract, chitosan succinamide, cinchona extract, *Gardenia jasminoides* Ellis extract, *Sasa veitchii* extract, *Sophora flavescens* Aiton extract, cape aloe extract, gentian extract, kojic acid, burdock (*Arctum lappa*) extract, fermented rice bran extract, rice germ oil, *Symphytum officinale* leaf extract, *Crataegus cuneata* fruit extract, rehmannia root extract, *Lithospermum officinale* root extract, perilla extract, *Tilia japonica* extract, peony root extract, Japanese white birch bark extract, silk extract, *Lonicera japonica* extract, *Equisetum arvense* extract, *Hedera helix* extract, sage (*Salvia officinalis*) leaf extract, *Malva sylvestris* leaf extract, *Swertia japonica* extract, mulberry bark extract, soybean seed extract, fermented soybean extract, tea extract, *Eugenia caryophyllata* flower extract, dexamethasone, *Angelica actiloba* root extract, *Calendula officinalis* flower extract, *Houttuynia cordata* extract, *Rosa multiflora* fruit extract, *Isodonis japonicus* extract, loquat (*Eriobotrya japonica*) extract, *Poria sclerotium* extract, *Vitis vinifera* leaf extract, placenta extract, bearberry (*Arctostaphylos uvaursi*) extract, *Luffa cylindrica* fruit extract, *Carthamus tinctorius* flower extract, *Paeonia suffruticosa* root extract, hop (*Humulus lupulus*) extract, *Aesculus hippocastanum* extract, *Sapindus mukurossi* peel extract, mucopolysaccharide, *Prunus persica* leaf extract, eucalyptus oil, *Saxifraga stolinefera* extract, Japanese mugwort (*Artemisia princeps* Pampan) extract, lactoferrin, lavender (*Lavendula angustifolia*) extract, *Lichi chienesis* extract, lemon extract, *Rosemarinus officinalis* extract, royal jelly extract, logwood *(Haematoxylon campechianum)* extract, *Sanguisorba officinalis* extract, hydrolyzed elastin, hydrolyzed conchiolin, hydrolyzed silk, hydrolyzed yeast, hydrolyzed yeast extract, Phaeophyceae extract, licorice extract, licorice flavonoid and perilla extract; metabolism stimulators such as coenzyme Q10; and the like.

Further, in the volatile substance-supporting porous silica of this embodiment, the same additives as in the first embodiment may be properly blended and processed as occasion demands.

The additives may be supported to the porous silica at the same time as the volatile substance or separately from the volatile substance.

Also, the volatile substance-supporting porous silica of this embodiment can be prepared in the same manner as in the first embodiment.

The form of the volatile substance-supporting porous silica of this embodiment is not particularly limited, and includes powder, granule, sheet, bulk, film and the like forms.

The volatile substance-supporting porous silica of this embodiment has the features of showing excellent adsorbability of the volatile substance and even milder desorbability by a physical or chemical stimulus from external. Therefore, the volatile substance-supporting porous silica of this embodiment can sustain a cooling effect, so that the volatile substance-supporting porous silica can be used for various manufactured articles. In addition, since the volatile substance is previously supported to the porous silica, when the volatile substance-supporting porous silica is applied to various manufactured articles, the pores thereof are inhibited from being clogged, and the volatile substances are vaporized earlier than water, so that the vaporization rate of water can be increased or maintained due to capillary phenomenon.

Accordingly, in one embodiment of this embodiment, a volatile substance-containing composition comprising the volatile substance-supporting porous silica of this embodiment is further provided. The volatile substance-containing composition of this embodiment is preferably pharmaceuticals and cosmetics.

The pharmaceuticals include external medicaments or quasi drugs for skin having forms of gel, jell, sol or ointment, which are used for adhesive preparations, cataplasms, packs, cooling gel sheets for fever or inflammation, cold compresses, electrically conductive pads for electrical therapeutic instruments and the like. Among them, the cooling gel sheets for fever or inflammation, icing agents for suppressing inflammation of muscles after exercises and cold compresses are preferable, and cooling gel sheets are more preferable. The cosmetics include make-up powders, lip creams, antiperspirants and the like.

The volatile substance-containing composition of this embodiment can be prepared in the same manner as a conventional method, except that the volatile substance-supporting porous silica of this embodiment is used; and the timing and method for the addition of the volatile substance-supporting porous silica are not limited as long as a composition which exhibits the desired effect of this embodiment is obtained. When a volatile substance-containing composition is prepared using the volatile substance-supporting porous silica that does not contain an emulsifying agent, it is preferable that in addition to the porous silica, an emulsifying agent is further added thereto, to prepare the volatile substance-containing composition in the same manner as a conventional method. For example, in the case of the adhesive preparation, the adhesive preparation can be prepared by adding the volatile substance-supporting porous silica to a substrate containing one or two or more kinds appropriately selected from resins, plasticizers, gelling agents, oils and fats, water and the like, and dispersing the mixture. Alternatively, the adhesive preparation can be obtained by applying the porous silica onto a substrate made of fabric or nonwoven fabric, and covering the substrate with facing materials such as polyethylene film as occasion demands. Here, the resin includes, for example, tackifiers such as rosin-based resins, polyterpene resins, cumarone-indene resins, petroleum resins and terpenephenol resins, and the like. The plasticizer includes liquid polybutenes, mineral oils, lanoline, liquid polyisoprenes, liquid polyacrylates, latexes and the like. The gelling agent includes, for example, agar, carboxymethyl cellulose, gelatin, furcellaran, sodium alginate, pectin, guar gum, tamarind gum, locust bean gum, xanthan gum, carrageenan, soybean polysaccharide, other inorganic gels or inorganic sols, inorganic salts, and the like.

The content of the volatile substance-supporting porous silica in the volatile substance-containing composition of this embodiment can be appropriately selected depending upon the manufactured articles used and purposes without particular limitations. For example, in the case of cooling gel sheets, the content is preferably from 0.5 to 20 parts by weight, and more preferably from 1 to 5 parts by weight, based on 100 parts by weight of a total amount of the raw material cooling gel sheet, from the viewpoint of improving the cooling effect by the volatile substance-supporting porous silica of this embodiment.

### (Third Embodiment)

Next, a third embodiment in which a substance to be supported is a thermal substance will be explained. In this embodiment, a thermal substance-supporting porous silica capable of sustaining a thermal feel for a long period of time and suppressing irritation to skin caused by the thermal substance, and a composition comprising the porous silica are provided.

The thermal substance in this embodiment includes capsicum extract, capsaicin, ginger extract, shogaol, vanillylamide nonylate, camphor, benzyl nicotinate, nicotinic acid amide, methyl salicylate and the like. These thermal substances can be used alone or in admixture of two or more kinds . As the thermal substance, it is preferable to use the capsicum extract alone or in combination of the capsicum extract with a different thermal substance from the viewpoint of the effects.

The average pore size, pore structure, pore volume, specific surface area and average particle size of the porous silica in this embodiment are the same as those in the first embodiment.

The porous silica in this embodiment can be prepared in the same manner as in the first embodiment. Also, the thermal substance can be supported to the porous silica in the same manner as in the first embodiment.

The content of the thermal substance is not particularly limited, and is preferably from 0.01 to 50 parts by weight, more preferably from 1 to 40 parts by weight, and even more preferably from 20 to 40 parts by weight, based on 100 parts by weight (on a solid basis) of the porous silica, from the viewpoint of releasing sustainability, lowered irritation and reduction in costs,

Further, it is preferable that in the thermal substance-supporting porous silica of this embodiment, an emulsifying agent is contained, from the viewpoint of improving sustainability of thermal feel, lowered irritation and dispersibility in water.

The emulsifying agent in this embodiment is the same as that in the first embodiment.

The timing and the method of the addition of the emulsifying agent when preparing the thermal substance-supporting porous silica of this embodiment are the same as those in the first embodiment.

Further, the thermal substance-supporting porous silica of this embodiment may contain, besides the above-mentioned thermal substance, a pharmaceutical, a functional substance or a metabolism accelerator in the same manner as in the second embodiment.

Furthermore, in the thermal substance-supporting porous silica of this embodiment, the same additives as in the first embodiment may be properly blended and processed as occasion demands.

The additives may be supported to the porous silica at the same time as the thermal substance or separately from the thermal substance.

Also, the thermal substance-supporting porous silica of this embodiment can be prepared in the same manner as in the first embodiment.

The form of the thermal substance-supporting porous silica of this embodiment is not particularly limited, and includes powder, granule, sheet, bulk, film and the like forms.

The thermal substance-supporting porous silica of this embodiment has the features of showing excellent adsorbability of the thermal substance and even milder desorbability by a physical or chemical stimulus from external. Therefore, the thermal substance-supporting porous silica of this embodiment can sustain a thermal effect, so that the thermal substance-supporting porous silica can be used for various manufactured articles.

Accordingly, in one embodiment of this embodiment, a thermal substance-containing composition comprising the thermal substance-supporting porous silica of this embodiment is further provided. The thermal substance-containing composition of this embodiment is preferably pharmaceuticals and cosmetics.

The pharmaceuticals include external medicaments for skin and quasi drugs therefor in the forms of gel, jell, sol or ointment, which are used for adhesive preparations, cream agents, cataplasms, packs, thermal gel sheets, compresses, electrically conductive pads for electrical therapeutic instruments and the like. Among them, warm compresses used for shoulder stiffness or lower backache and hand creams used for accelerating blood circulation in finger tips are preferable, warm compresses even more preferable. The cosmetics include make-up powders, lip creams and the like.

The thermal substance-containing composition of this embodiment can be prepared in the same manner as a conventional method, except that the thermal substance-supporting porous silica of this embodiment is used; and the timing and method for the addition of the thermal substance-supporting porous silica are not limited as long as a composition which exhibits the desired effect of this embodiment is obtained. When a thermal substance-containing composition is prepared using the thermal substance-supporting porous silica that does not contain an emulsifying agent, it is preferable that in addition to the porous silica, an emulsifying agent is further added thereto, to prepare the thermal substance-containing composition in the same manner as a conventional method. For example, in the case of the adhesive preparation, the adhesive preparation can be prepared in the same manner as in the adhesive preparation exemplified in the second embodiment except that the thermal substance is used in place of the volatile substance in the second embodiment.

The content of the thermal substance-supporting porous silica in the thermal substance-containing composition of this embodiment can be appropriately selected depending upon manufactured articles used and purposes without particular limitations. For example, in the case of a warm compress, the content is preferably from 0.1 to 30 parts by weight, and more preferably from 0.5 to 10 parts by weight, based on 100 parts by weight of a total amount of the raw material warm compress, from the viewpoint of improving the thermal effect and its sustainability by the thermal substance-supporting porous silica of this embodiment.

### (Fourth Embodiment)

Next, a fourth embodiment in which a substance to be supported is a plant polyphenol will be explained. In this embodiment, a plant polyphenol-supporting porous silica sustaining a deodorizing effect and a composition comprising the porous silica are provided.

The plant polyphenol in this embodiment is a substance contained in most of plants in which photosynthesis is carried out. The plant used as its raw material is not particularly limited. For example, the plant includes plants belonging to Theaceae such as tea; plants belonging to Vitaceae such as grape; plants belonging to Rubiaceae such as coffee; plants belonging to Sterculiaceae such as cacao; plants belonging to Polygonaceae such as buckwheat; plants belonging to Saxifragaceae such as gooseberry *(Ribes grossularia),* blackcurrant *(Ribes nigrum),* and redcurrant *(Ribes rubrum);* plants belonging to Ericaceae such as blueberry, whortleberry (*Vaccinium myrtillus* LINNE), black huckleberry (*Gaylussacia baccata* C. KOCH), cranberry (*Vaccinium macrocarpon* Ait) and mountain cranberry (*Vaccinium vitis-idea*); plants belonging to Gramineae such as red rice (*Oryza sativa* subsp. *japonica*) and purple corn (Zea mays LINNE); plants belonging to Moraceae such as mulberry (*Morus bombycis*); plants belonging to Caprifoliaceae such as elderberry *(Sambucus sieboldiana)* and blue honeysuckle (*Lonicera caerulea var. emphyllocalyx*); plants belonging to Rosaceae such as plum *(Prunus domestica),* European blackberry *(Rubus fruticosus* Agg.), loganberry (*Rubus loganobaccus*), salmonberry (*Rubus spectabilis* Pursh), wild red raspberry (*Rubus idaeus* L. subsp. *melanolasius* Focke), *Rubus idaeus* LINNE, *Rubus caesius* LINNE, garden strawberry (*Fragaria Xananassa*), black raspberry (*Rubus occidentalis*), Morello cherry (*Prunus cerasus* LINNE var. *austere* LINNE), Yoshino cherry (*Prunus yedoensis*), sweet cherry (*Prunus avium*), sweet tea (*Rubus suavissimus*) and apple (*Malus pumila*); plants belonging to Leguminosae such as Japanese pagoda tree (*Sophora japonica*), adzuki bean (*Vigna angularis*), soybean, tamarind (*Tamarindus indica*), mimosa and catechu (*Acacia catechu* WILLD.); plants belonging to Dioscoreaceae such as winged yam (*Dioscorea alata* L.); plants belonging to Ebenaceae such as persimmon *(Diospyros kaki);* plants belonging to Compositae such as Japanese mugwort (*Artemisia princeps* Pampan) and garland chrysanthemum *(Chrysanthemum coronarium);* plants belonging to Musaceae such as banana (*Musa paradisiacal var. sapientum*); plants belonging to Convolvulaceae such as Yawaraka purple sweet potato; plants belonging to Malvaceae such as roselle (*Hibiscus sabdariffa*); plants belonging to Labitae such as red perilla (*Perilla frutescens var. acuta*); and plants belonging to Cruciferae such as red cabbage, and the sites can be arbitrarily selected from as fruits, fruit skins, flowers, leaves, stems, barks, roots, tuberous roots, seeds, seed skins and the like depending upon on the kinds of the plant.

The plant polyphenol can be obtained by extraction from the above-mentioned plant with a solvent such as hot water, ethyl acetate, methanol, ethanol or isopropanol. Among them, the plant polyphenol extracted from tea leaves, a plant belonging to Theaceae is preferable. Specific substances thereof include catechin, gallocatechin, gallocatechin gallate, epicatechin, epicatechin gallate, epigallocatechin, epigallocatechin gallate, theaflavin and the like, including one or a mixture of two or more kinds selected therefrom. Also, the purity of the plant polyphenol in the extract is not particularly limited, and the purity is preferably 40% or more, and more preferably 60% or more. Here, a commercially available plant polyphenol-containing material such as SUNFLAVON (manufactured by Taiyo Kagaku Co., Ltd), TEAFLAN (manufactured by ITO EN LTD.), SUNOOLONG (manufactured by Suntory, Limited), or POLYPHENON (manufactured by Tokyo Food Techno Co., Ltd.) can be used.

The average pore size, pore structure, pore volume, specific surface area and average particle size of the porous silica in this embodiment are the same as those in the first embodiment.

The porous silica in this embodiment can be prepared in the same manner as in the first embodiment. Also, the plant polyphenol can be supported to the porous silica in the same manner as in the first embodiment.

The content of the plant polyphenol is not particularly limited, and is preferably from 10 to 1000 parts by weight, more preferably from 20 to 500 parts by weight, and even more preferably from 50 to 200 parts by weight, based on 100 parts by weight (on a solid basis) of the porous silica, from the viewpoint of sustainability of the deodorizing effect and reduction in costs.

Further, it is preferable that in the plant polyphenol-supporting porous silica of this embodiment, an emulsifying agent is contained, from the viewpoint of improving sustainability of deodorizing effect and dispersibility in water.

The emulsifying agent in this embodiment is the same as that in the first embodiment.

The timing and the method of the addition of the emulsifying agent when preparing the plant polyphenol-supporting porous silica of this embodiment are the same as those in the first embodiment.

Also, the plant polyphenol-supporting porous silica of this embodiment can be prepared in the same manner as in the first embodiment.

The form of the plant polyphenol-supporting porous silica of this embodiment is not particularly limited, and includes powder, granule, sheet, bulk, film and the like forms.

The plant polyphenol-supporting porous silica of this embodiment has the features of having excellent adsorbability of the plant polyphenol and protecting the plant polyphenol from a physical or chemical stimulus from external. Therefore, the plant polyphenol-supporting porous silica of this embodiment can sustain a deodorizing effect, so that the plant polyphenol-supporting porous silica can be applied to various manufactured articles.

Accordingly, in one embodiment of this embodiment, a plant polyphenol-containing composition comprising the plant polyphenol-supporting porous silica of this embodiment is further provided. The plant polyphenol-containing composition of this embodiment is preferably a filter for cleaning air which can be used for air conditioners, air cleaners, kerosene fan heaters, humidifiers, dehumidifier, vacuum cleaners, masks and the like.

The plant polyphenol-containing composition of this embodiment can be prepared in the same manner as a conventional method, except that the plant polyphenol-supporting porous silica of this embodiment is used; and the timing and method for the addition of the plant polyphenol-supporting porous silica are not limited as long as a composition which exhibits the desired effect of this embodiment is obtained. When a plant polyphenol-containing composition is prepared using the plant polyphenol-supporting porous silica that does not contain an emulsifying agent, it is preferable that in addition to the porous silica, an emulsifying agent is further added thereto, to prepare the plant polyphenol-containing composition in the same manner as a conventional method.

When the plant polyphenol-containing composition of this embodiment is, for example, a filter, the filter can be produced by supporting the plant polyphenol-supporting porous silica of this embodiment on a substrate with air-permeability. In this case, the water content of the composition containing a plant polyphenol-supporting porous silica is not particularly limited, and is preferably from 1 to 15 % by weight, and more preferably from 3 to 15 % by weight, from the viewpoint of sustaining a deodorizing effect.

The substrate for the filter includes fabrics, nonwoven fabrics and processed products of nonwoven fabrics, nets, and sponges, and commonly used thermoplastic films and thin plates such as polyethylene film, polypropylene film and polyester film, papers and paper processed products such as those having corrugated or honeycomb structures, metal sheets and nets and ceramic substrates and processed products thereof. Among them, the sheet such as a film or thin plate which is poor in air permeability may be used as an air-permeable filter by producing fine holes on the sheet to improve its air permeability, or one assembled into an air-permeable shape such as corrugated core or honeycomb forms may be used. Among these filter substrates, especially when the nonwoven fabric or the like is used, not only a relatively uniform air permeability can be secured but also working of encapsulation is facilitated, so that such filters are more advantageously used.

In the former case among them where the substrate itself is made from a substrate having air-permeability, from the aspect of the materials, a substrate formed from materials having good affinity to both the components constituting the antibacterial deodorant is suitably used, in order to excellently keep the antibacterial deodorant of this embodiment. The material includes polymer materials having an anionic functional group (such as carboxyl group and sulfonate group), cellulose-based fibers and the like. On the other hand, from the aspect of structures thereof, those having bulky structure such that a large amount of the antibacterial deodorant can be held are preferably used. As an alternative, when an electret nonwoven fabric having a function of capturing fine particles of bacteria, dusts and the like floating in air by its own electrostatic force is used as a substrate, highly efficient capturing, antibacterial and deodorizing effects can be expected with low pressure drop. As the electret nonwoven fabric, an electret melt-blown nonwoven fabric or an electret split-fiber nonwoven fabric, having especially high capturing efficiency is preferable.

In order to support the porous silica of this embodiment to the above-mentioned substrate, for example, a solution containing the porous silica may be applied to the substrate, or the substrate is dipped in the above-mentioned solution and then drawn up. Alternatively, a filter may be directly formed by making paper from the above-mentioned solution together with the pulp to include the porous silica in the filter.

Here, to the solution, there may added a binder which is applied to or impregnated into the substrate that functions to solidify or cure the binder thereby forming a coating film containing the porous silica on the surface of the substrate or on the surface of the fibers such as paper constituting the substrate or the like, or functions to firmly bind pulps in a state that the antibacterial deodorant is incorporated into the substrate during the production of filter.

The binder includes water-soluble or emulsive synthetic resins such as acrylic resin, acrylic-silicone resin, acrylic-urethane resin, urethane resin, water-soluble epoxy resin, aqueous vinyl urethane resin, and air drying fluororesin; natural resins such as shellac resin, gum copal, and gum dammar; inorganic binders such as colloidal silica; and organic-inorganic complex binders such as complex of polyisocyanate and colloidal silica. The amount of the binder is not particularly limited, and is preferably from 5 to 150 parts by weight, and more preferably from 20 to 70 parts by weight, based on 100 parts by weight of the porous silica or the plant polyphenol-supporting porous silica, from the viewpoint of sufficiently obtaining an effect of forming a coating film and sustaining deodorizing effect.

The content of the plant polyphenol-supporting porous silica in the plant polyphenol-containing composition of this embodiment can be appropriately selected depending upon manufactured articles used and purposes without particular limitations.

### (Fifth Embodiment)

Next, a fifth embodiment in which a substance to be supported is an organic colorant will be explained. In this embodiment, since the release of the organic colorant can be controlled for a long period of time, an organic colorant-supporting porous silica having excellent water resistance, light fastness and color development, and a composition containing said porous silica are provided.

The organic colorant in this embodiment includes acid dyes, basic dyes, vat dyes, direct dyes, oil-soluble dyes, reactive dyes, organic pigments, natural pigments and the like.

The acid dye is not particularly limited. The acid dye includes, for example, C.I. Acid Orange 7, C.I. Acid Orange 19, C.I. Acid Violet 49, C.I. Acid Black 2, C.I. Acid Black 7, C.I. Acid Black 24, C.I. Acid Black 26, C.I. Acid Black 31, C.I. Acid Black 52, C.I. Acid Black 63, C.I. Acid Black 112, C.I. Acid Black 118, C.I. Acid Blue 9, C.I. Acid Blue 22, C.I. Acid Blue 40, C.I. Acid Blue 59, C.I. Acid Blue 93, C.I. Acid Blue 102, C.I. Acid Blue 104, C.I. Acid Blue 113, C.I. Acid Blue 117, C.I. Acid Blue 120, C.I. Acid Blue 167, C.I. Acid Blue 229, C.I. Acid Blue 234, C.I. Acid Red 1, C.I. Acid Red 6, C.I. Acid Red 32, C.I. Acid Red 37, C.I. Acid Red 51, C.I. Acid Red 52, C.I. Acid Red 80, C.I. Acid Red 85, C.I. Acid Red 87, C.I. Acid Red 92, C.I. Acid Red 94, C.I. Acid Red 115, C.I. Acid Red 180, C.I. Acid Red 256, C.I. Acid Red 315, C.I. Acid Red 317, Brown No. 201, Yellow No. 4, Yellow No. 5, Yellow No. 202, Yellow No. 203, Yellow No. 402, Yellow No. 403, Yellow No. 406, Yellow No. 407, Black No. 401, Violet No. 401, Blue No. 1, Blue No. 2, Blue No. 202, Blue No. 203, Blue No. 205, Red No. 2, Red No. 3, Red No. 102, Red No. 104, Red No. 105, Red No. 106, Red No. 201, Red No. 227, Red No. 230, Red No. 231, Red No. 232, Red No. 401, Red No. 502, Red No. 503, Red No. 504, Red No. 506, Green No. 3, Green No. 201, Green No. 205, Green No. 401, Green No. 402, Orange No. 205, Orange No. 207 Orange No. 402 and the like.

The basic dye is not particularly limited. The basic dye includes, for example, C.I. Basic Yellow 11, C.I. Basic Yellow 28, C.I. Basic Violet 3, C.I. Basic Violet 7, C.I. Basic Violet 14, C.I. Basic Violet 27, C.I. Basic Black 2, C.I. Basic Blue 1, C.I. Basic Blue 3, C.I. Basic Blue 5, C.I. Basic Blue 7, C.I. Basic Blue 9, C.I. Basic Blue 24, C.I. Basic Blue 25, C.I. Basic Blue 26, C.I. Basic Blue 28, C.I. Basic Blue 29, C.I. Basic Red 1, C.I. Basic Red 2, C.I. Basic Red 9, C.I. Basic Red 12, C.I. Basic Red 13, C.I. Basic Red 14, C.I. Basic Red 37, Red No. 213, Red No. 214 and the like.

The vat dye is not particularly limited. The vat dye includes, for example, C.I. Vat Blue 1, Blue No. 201, Blue No. 204, Red No. 226 and the like.

The direct dye is not particularly limited. The direct dye includes, for example, C.I. Direct Yellow 11, C.I. Direct Yellow 12, C.I. Direct Yellow 17, C.I. Direct Yellow 23, C.I. Direct Yellow 25, C.I. Direct Yellow 29, C.I. Direct Yellow 42, C.I. Direct Yellow 61, C.I. Direct Yellow 71, C.I. Direct Orange 26, C.I. Direct Orange 34, C.I. Direct Orange 39, C.I. Direct Orange 44, C.I. Direct Orange 46, C.I. Direct Orange 60, C.I. Direct Green 59, C.I. Direct Violet 47, C.I. Direct Violet 48, C.I. Direct Violet 51, C.I. Direct Brown 109, C.I. Direct Black 17, C.I. Direct Black 19, C.I. Direct Black 32, C.I. Direct Black 51, C.I. Direct Black 71, C.I. Direct Black 108, C.I. Direct Black 146, C.I. Direct Black 154, C.I. Direct Black 166, C.I. Direct Blue 1, C.I. Direct Blue 6, C.I. Direct Blue 22, C.I. Direct Blue 25, C.I. Direct Blue 71, C.I. Direct Blue 86, C.I. Direct Blue 90, C.I. Direct Blue 106, C.I. Direct Blue 203, C.I. Direct Blue 264, C.I. Direct Red 1, C.I. Direct Red 4, C.I. Direct Red 17, C.I. Direct Red 23, C.I. Direct Red 28, C.I. Direct Red 31, C.I. Direct Red 37, C.I. Direct Red 80, C.I. Direct Red 81, C.I. Direct Red 83, C.I. Direct Red 201, C.I. Direct Red 227, C.I. Direct Red 242 and the like.

The oil-soluble dye is not particularly limited. The oil-soluble dye includes, for example, Yellow No. 201, Yellow No. 204, Yellow No. 404, Yellow No. 405, Violet No. 201, Blue No. 403, Red No. 215, Red No. 218, Red No. 223, Red No. 225, Red No. 501, Red No. 505, Green No. 202, Green No. 204, Orange No. 201, Orange No. 206, Orange No. 403 and the like.

The reactive dye is not particularly limited. The reactive dye includes, for example, C.I. Reactive Orange 16, C.I. Reactive Black 5, C.I. Reactive Blue 21, C.I. Reactive Blue 27, C.I. Reactive Blue 28, C.I. Reactive Blue 38, C.I. Reactive Red 21 and the like.

The organic pigment is not particularly limited. The organic pigment includes, for example, C.I. Pigment Yellow 14, C.I. Pigment Yellow 83, C.I. Pigment Green 7, C.I. Pigment Violet 19, C.I. Pigment Violet 23, C.I. Pigment Blue 27, C.I. Pigment Red 166, Yellow No. 205, Yellow No. 401, Blue No. 404, Red No. 201, Red No. 202, Red No. 203, Red No. 204, Red No. 205, Red No. 206, Red No. 207, Red No. 208, Red No. 219, Red No. 220, Red No. 221, Red No. 228, Red No. 404, Red No. 405, Orange No. 203, Orange No. 204 Orange No. 401 and the like.

The natural pigment includes, for example, chlorophyll, β-carotene, lutein, lycopene, *Gardenia jasminoides* yellow pigment, *Carthamus tinctorius* yellow pigment, turmeric pigment, ang-khak yellow pigment, palm oil carotene, ang-khak pigment, *Gardenia jasminoides* red pigment, *Carthamus tinctorius* red pigment, beet red, cochineal pigment, lac pigment, madder pigment, perilla pigment, red cabbage pigment, red daikon pigment, purple sweet potato pigment, purple corn pigment, grape skin pigment, grape juice pigment, blueberry pigment, elderberry pigment, capsicum pigment, annatto pigment, *Gardenia jasminoides* blue, *Gardenia jasminoides* yellow, *Carthamus tinctorius* yellow, ang-khak yellow, Spirulina pigment, phycocyanin, cacao pigment, Japanese persimmon pigment and the like.

The above-mentioned organic colorant can be used alone or in a combination of two or more kinds. Among them, the dyes which have excellent color development are preferable. When used as an ink, especially an inkjet ink, one or more members selected from the group consisting of C.I. Acid Blue 9, C.I. Acid Blue 22, C.I. Acid Blue 40, C.I. Acid Blue 59, C.I. Acid Blue 93, C.I. Acid Blue 102, C.I. Acid Blue 104, C.I. Acid Blue 113, C.I. Acid Blue 117, C.I. Acid Blue 120, C.I. Acid Blue 167, C.I. Acid Blue 229, C.I. Acid Blue 234, C.I. Acid Red 1, C.I. Acid Red 6, C.I. Acid Red 32, C.I. Acid Red 37, C.I. Acid Red 51, C.I. Acid Red 52, C.I. Acid Red 80, C.I. Acid Red 85, C.I. Acid Red 87, C.I. Acid Red 92, C.I. Acid Red 94, C.I. Acid Red 115, C.I. Acid Red 180, C.I. Acid Red 256, C.I. Acid Red 289, C.I. Acid Red 315, C.I. Acid Red 317 and the like.

The average pore size, pore structure, pore volume and specific surface area of the porous silica in this embodiment are the same as those in the first embodiment.

The average particle size of the porous silica in this embodiment is preferably from 50 nm to 10 µm, more preferably from 50 nm to 5 µm, even more preferably from 50 to 500 nm, and still even more preferably from 50 to 300 nm, from the viewpoint of stability of the organic colorant.

The porous silica of this embodiment can be prepared in the same manner as in the first embodiment. Also, the organic colorant can be supported to the porous silica in the same manner as in the first embodiment.

The content of the organic colorant is not particularly limited, and is preferably from 0.01 to 300 parts by weight, more preferably from 5 to 100 parts by weight, and even more preferably from 5 to 50 parts by weight, based on 100 parts by weight (on a solid basis) of the porous silica, from the viewpoint of stability and reduction in costs.

Further, it is preferable that in the organic colorant-supporting porous silica of this embodiment, an emulsifying agent is contained, from the viewpoint of improving stability, release-controlling ability and dispersibility in water.

The emulsifying agent in this embodiment are the same as those in the first embodiment. The content of the emulsifying agent is preferably from 0.01 to 80 parts by weight, more preferably from 0.01 to 50 parts by weight, even more preferably from 1 to 50 parts by weight, and still even more preferably from 1 to 20 parts by weight, based on 100 parts by weight (on a solid basis) of the organic colorant-supporting porous silica, from the viewpoint of improving color development and dispersibility in water.

The timing and the method of the addition of the emulsifying agent when preparing the organic colorant-supporting porous silica of this embodiment are the same as those in the first embodiment.

Further, in the organic colorant-supporting porous silica of this embodiment, the same additives as in the first embodiment may be properly blended and processed as occasion demands.

The additives may be supported to the porous silica at the same time as the organic colorant or separately from the organic colorant.

Also, the organic colorant-supporting porous silica of this embodiment can be prepared in the same manner as in the first embodiment.

The form of the organic colorant-supporting porous silica of this embodiment is not particularly limited, and includes powder, granule, sheet, bulk, film and the like forms.

The organic colorant-supporting porous silica of this embodiment has the features of excellent water resistance, light fastness and color development, and also has the feature of being stable to a physical or chemical stimulus from external. Therefore, the porous silica of this embodiment can sustain excellent color development for a long period of time, so that the organic colorant-supporting porous silica can be used for various products such as pigment preparations for inks, foodstuff and cosmetics. The ink includes inkjet inks, inks for ball point pens and the like. The foodstuff include snacks and candies such as chewing gum, candies, tablet sweets, gummi candies, chocolates, biscuits and snacks; frozen desserts such as ice creams, sherbets and ice candies; refreshing drinks, carbonated beverages, luxury beverages and the like. The cosmetics include make-up powders, lip creams and the like. Among them, inkjet inks which are most highly desired to have water resistance, light fastness and color development are preferable.

The organic colorant-containing composition of this embodiment can be prepared in the same manner as a conventional method, except that the organic colorant-supporting porous silica of this embodiment is used; and the timing and the method of the addition of the organic colorant-supporting porous silica are not limited as long as a composition which exhibits the desired effects of this embodiment is obtained. When an organic colorant-containing composition is prepared using the organic colorant-supporting porous silica that does not contain an emulsifying agent, it is preferable in addition to the porous silica, an emulsifying agent is further added thereto, to prepare the organic colorant-containing composition in the same manner as a conventional method.

The content of the organic colorant-supporting porous silica in the organic colorant-containing composition of this embodiment is not particularly limited, and can be appropriately selected depending upon the manufactured articles used and the purposes. For example, in the case of inkjet inks, the content is preferably from 0.01 to 90 parts by weight, more preferably from 1 to 60 parts by weight, and even more preferably from 10 to 60 parts by weight, based on 100 parts by weight of the entire amount of the raw material inkjet ink, from the viewpoint of color development and reduction in costs.

In another embodiment of the present invention, besides the composition containing the above-mentioned volatile substance-supporting porous silica, a high cooling effect and a high sustainability can be obtained by compositions containing the porous silica that do not support a volatile substance.

### EXAMPLES

The present invention will be described more specifically by means of the examples, without intending to limit the present invention to the following

### examples.

The form of pores was determined with a fully automatic X-ray diffractometer (RINT ULTIMA II, manufactured by Rigaku Denki K.K.). The average pore size, pore volume and specific surface area were obtained from the nitrogen adsorption isotherm determined according to a known BET method. The average particle size was determined with a laser diffraction particle size distribution analyzer (manufactured by HELOS & RODOS SYMPATEC).

### Preparation Example 1 of Porous Silica

Fifty grams of No. 1 sodium silicate (SiO₂/Na₂O = 2.00) manufactured by Nippon Chemical Industrial Co., LTD. was dispersed in 1000 ml of a 0.1 M aqueous solution of octadecyltrimethylammonium chloride [C₁₈H₃₇N(CH₃)₃Cl], a surfactant, and the dispersion was heated at 70°C for 3 hours while stirring. Thereafter, while heating the mixture at 70°C and stirring, a 2 N hydrochloric acid was added to the dispersion to lower its pH to 8.5, and the mixture was further heated at 70°C for 3 hours while stirring. A solid product was temporarily filtered and re-dispersed in 1000 ml of ion-exchanged water while stirring. The procedures of filtration and dispersion-stirring were repeated 5 times, and thereafter the residue was dried at 40°C for 24 hours. The dried solid product was heated in nitrogen gas at 450°C for 3 hours, and thereafter the heated solid product was baked in air at 550°C for 6 hours to give a porous silica A. It was confirmed by X-ray diffraction that pores having a hexagonal structure were formed in the resulting porous silica A. In addition, the porous silica A had an average pore size of 3.3 nm, a specific surface area of 941 m²/g and a pore volume of 1.13 cm³/g.

### Preparation Example 2 of Porous Silica

Powder sodium silicate (SiO₂/Na₂O = 2.00) manufactured by Nippon Chemical Industrial Co., LTD. was baked in air at 700°C for 6 hours to give crystals of δ-Na₂Si₂O₅. Fifty grams of the crystals obtained were dispersed in 500 ml of ion-exchanged water, and the dispersion was stirred at 25°C for 3 hours. Thereafter, a solid content was collected by filtration to give 50 g (on a dry basis) of wet kanemite, a layered silicate. This kanemite, without being dried, was dispersed in 1000 ml of a 0.1 M sodium oleylsulfate, a surfactant, and the dispersion was heated at 70°C for 3 hours while stirring. Thereafter, a 2 N hydrochloric acid was added to the dispersion while heating at 70°C and stirring to lower its pH to 8.5, and the mixture was further heated at 70°C for 3 hours while stirring. A solid product was temporarily filtered and re-dispersed in 1000 ml of ion-exchanged water while stirring. The procedures of filtration and dispersion-stirring were repeated 5 times, and thereafter the residue was dried at 40°C for 24 hours. A dried solid product was heated in nitrogen gas at 450°C for 3 hours, and thereafter the heated solid product was baked in air at 550°C for 6 hours to give a porous silica B containing silicon dioxide. It was confirmed by X-ray diffraction that pores having a hexagonal structure were formed in the resulting porous silica B. In addition, the porous silica B had an average pore size of 2.9 nm, a specific surface area of 932 m²/g, a pore volume of 1.09 cm³/g, and an average particle size of 350 nm.

### Preparation Example 3 of Porous Silica

Two grams of polyethylene glycol, 15 g of ion-exchanged water and 60 ml of a 2 N hydrochloric acid were dispersed at 80°C while stirring. Thereafter, 4.25 g of tetraethoxysilane (TEOS) was added to the dispersion, and the mixture was stirred at 80°C for 12 hours. A solid product was temporarily filtered and re-dispersed in 1000 ml of ion-exchanged water while stirring. The procedures of filtration and dispersion-stirring were repeated 5 times, and thereafter the residue was dried at 40°C for 24 hours. The dried solid product was heated in nitrogen gas at 450°C for 3 hours, and thereafter the heated solid product was baked in air at 550°C for 6 hours to give a porous silica C containing silicon dioxide. It was confirmed by X-ray diffraction that pores having a hexagonal structure were formed in the resulting porous silica C. In addition, the porous silica C had an average pore size of 2.8 nm, a specific surface area of 928 m²/g, a pore volume of 1.02 cm³/g, and an average particle size of 300 nm.

### Preparation Example 4 of Porous Silica

One-hundred grams of a cetyltrimethyl hydroxide (CTMA) solution prepared by contacting a 29% by weight solution of N,N,N-trimethyl-1-hexadecylammonium chloride with a hydroxide-halide exchange resin was mixed with 100 g of an aqueous solution of tetramethylammonium (TMA) silicate (silica 10%) while stirring. Thereto was added 25 g of HiSil, a sedimentary hydrated silica containing about 6% by weight of free water and about 4.5% by weight of hydrated bound water and having a cut particle size of 0.02 µm. The mixture obtained was reacted at 90°C for 1 day. The resulting solid product was collected by filtration, and the residue was dried at 40°C. Next, the product was baked in nitrogen at 540°C for 1 hour, and then in air for 6 hours to give a porous silica D containing silicon dioxide. It was confirmed by X-ray diffraction that pores having a hexagonal structure were formed in the resulting porous silica D. In addition, the porous silica D had an average pore size of 3.9 nm, a specific surface area of 945 m²/g, a pore volume of 1.15 cm³/g, and an average particle size of 1.1 µm.

### Preparation Example 5 of Porous Silica

Two grams of sodium laurylaminopropionate, 15 g of ion-exchanged water and 60 ml of a 2 N hydrochloric acid were dispersed at 80°C while stirring. Thereafter, 4.25 g of tetraethoxysilane (TEOS) was added to the dispersion, and the mixture was stirred at 80°C for 12 hours. A solid product was temporarily filtered and re-dispersed in 1000 ml of ion-exchanged water while stirring. The procedures of filtration and dispersion-stirring were repeated 5 times, and thereafter the residue was dried at 40°C for 24 hours. The dried solid product was heated in nitrogen gas at 450°C for 3 hours, and thereafter the heated solid product was baked in air at 550°C for 6 hours to give a porous silica E containing silicon dioxide. It was confirmed by X-ray diffraction that pores having a hexagonal structure were formed in the resulting porous silica E. In addition, the porous silica E had an average pore size of 3.9 nm, a specific surface area of 945 m²/g, a pore volume of 1.15 cm³/g, and an average particle size of 5.1 µm.

### Preparation Example 6 of Porous Silica

Fifty grams of No. 1 sodium silicate (SiO₂/Na₂O = 2.00) manufactured by Nippon Chemical Industrial Co., LTD. was dispersed in 1000 ml of a 0.1 M aqueous solution of octadecyltrimethylammonium chloride [C₁₈H₃₇N(CH₃)₃Cl], a surfactant, and the dispersion was heated at 70°C for 3 hours while stirring. Thereafter, a 2 N hydrochloric acid was added to the dispersion while heating and stirring at 70°C to lower its pH to 8.5, and then further heated while stirring at 70°C for 3 hours. A solid product was temporarily filtered and re-dispersed in 1000 ml of ion-exchanged water while stirring. The procedures of filtration and dispersion-stirring were repeated 5 times, and the residue obtained was dried at 40°C for 24 hours. The dried solid product was heated in nitrogen gas at 450°C for 3 hours, and the heated solid product was baked in air at 550°C for 6 hours to obtain a porous silica F. It was confirmed by X-ray diffraction that pores having a hexagonal structure were formed in the resulting porous silica F. In addition, the porous silica F had an average pore size of 2.7 nm, a specific surface area of 941 m²/g, a pore volume of 1.13 cm³/g, and an average particle size of 380 nm.

### Preparation Example 7 of Porous Silica

Ten grams of the porous silica F obtained in Preparation Example 6 was dispersed in 1000 ml of ultrapure water. Thereafter, 10 g of N-2-(aminomethyl)-3-aminopropylmethyldimethoxysilane was added to the dispersion, and the mixture was stirred at room temperature for 30 minutes. Thereafter, the liquid mixture was subjected to suction filtration, and the solid obtained was allowed to stand at 120°C for 1 hour to dryness. Thereafter, the dried solid was dispersed in 1000 ml of ultrapure water, and the dispersion was stirred for 10 minutes. The liquid mixture was further subjected to suction filtration, and a solid obtained was dried at 40°C for 48 hours to give a porous silica G. It was confirmed by X-ray diffraction that pores having a hexagonal structure were formed in the resulting porous silica G. In addition, the porous silica G had an average pore size of 2.9 nm, a specific surface area of 895 m²/g, and an average particle size of 495 nm.

### Preparation Example 8 of Porous Silica

Two grams of polyglycerol, 15 g of ion-exchanged water and 60 ml of a 2 N hydrochloric acid were dispersed at 80°C while stirring. Thereafter, 4.25 g of tetraethoxysilane (TEOS) was added to the dispersion, and thereafter the mixture was stirred at 80°C for 12 hours. A solid product was temporarily filtered and re-dispersed in 1000 ml of ion-exchanged water while stirring. The procedures of filtration and dispersion-stirring were repeated 5 times, and thereafter the residue obtained was dried at 40°C for 24 hours. A dried solid product was heated in nitrogen gas at 450°C for 3 hours, and thereafter the heated product was baked in air at 550°C for 6 hours to give a porous silica H containing silicon dioxide. It was confirmed by X-ray diffraction that pores having a hexagonal structure were formed in the resulting porous silica H. In addition, the porous silica H had an average pore size of 2.6 nm, a specific surface area of 913 m²/g, and a pore volume of 0.99 cm³/g.

### Example 1-1

A solution prepared by dissolving 14 g of natural L-menthol in 200 g of ethanol was added to 20 g of the porous silica A while mixing, and thereafter the mixture was stirred at 40°C for 30 minutes with a homomixer. Subsequently, the solvent (ethanol) was removed by concentration with a rotary evaporator to give 34 g of a menthol-supporting porous silica (water content: 2% by weight).

### Examples 1-2 to 1-5

The same procedures as in Example 1-1 were carried out except that 20 g of the porous silica B, C, D or E was used in place of the porous silica A to give each of menthol-supporting porous silicas.

### Example 1-6

Thirty grams of natural L-menthol was mixed with 70 g of the porous silica A. Thereafter, the mixture was placed in a tightly sealed container and allowed to stand at 40°C for 1 week under a reduced pressure. The sublimed menthol was allowed to adsorb to a porous silica containing silicon dioxide to give 100 g of a menthol-supporting porous silica.

### Example 1-7

A solution prepared by dissolving 1 g of a polyglycerol fatty acid ester (SUNSOFT AZ-18G, manufactured by Taiyo Kagaku Co., Ltd., HLB = 18) as an emulsifying agent in 10 g of ethanol was added to 20 g of the menthol-supporting porous silica obtained in Example 1-1 while mixing, and thereafter the solvent (ethanol) was removed by concentration with a rotary evaporator to give 20 g of a menthol-supporting porous silica.

### Example 1-8

A solution prepared by dissolving 10 g of a polyglycerol fatty acid ester (SUNSOFT AZ-18G, manufactured by Taiyo Kagaku Co., Ltd., HLB = 18) as an emulsifying agent in 30 g of ethanol was added to 20 g of the menthol-supporting porous silica obtained in Example 1-1 while mixing, and thereafter the solvent (ethanol) was removed by concentration with a rotary evaporator to give 26 g of a menthol-supporting porous silica.

### Example 1-9

A solution prepared by dissolving 1 g of a polyglycerol fatty acid ester (SUNSOFT AZ-18EG, manufactured by Taiyo Kagaku Co., Ltd., HLB = 18) as an emulsifying agent in 10 g of water was added to 20 g of the menthol-supporting porous silica obtained in Example 1-1 while mixing, to give 31 g of a menthol-supporting porous silica.

### Example 1-10

The same procedures as in Example 1-7 were carried out except that a sucrose fatty acid ester (RYOTO Sugar Ester, manufactured by Mitsubishi-Kagaku Foods Corporation, L-1695) was used in place of the polyglycerol fatty acid ester, to give 20 g of a menthol-supporting porous silica.

### Comparative Example 1-1

Five grams of β-cyclodextrin (Celdex N, manufactured by NIHON SHOKUHIN KAKO CO., LTD.) was dissolved in 66.2 g of water while stirring, and 10 g of natural L-menthol was added to the solution at a temperature of 50°C, and the mixture was homogenized at 1000 rpm/min. for 5 minutes to give an emulsion. The emulsion obtained was spray-dried under the conditions of a blast temperature of from 130° to 140°C, and an exhaust air temperature of from 60° to 70°C to give 12 g of a menthol-supporting porous silica.

### Comparative Example 1-2

The same procedures as in Comparative Example 1-1 were carried out except that an amount of water used was changed to 60 g, and that 40 g of a branched cyclodextrin (ISOELITE P, manufactured by ENSUIKO Sugar Refining Co., Ltd.) was used in place of the β-cyclodextrin to give 40 g of a menthol-supporting porous silica.

### Examples 1-A1 to 1-A10 and Comparative Examples 1-A1 and 1-A2

One gram of the menthol-supporting porous silica obtained in each Example or the menthol-supporting porous substance obtained in each Comparative Example was added to 20 g of natural chicle, 66.2 g of powdered sugar and 12 g of glucose syrup while mixing, and the mixture was mixed with a high-shear mixer at about 50°C according to a conventional method. The mixture was cooled and then subjected to extension molding with a roller to prepare 3 g per piece of a chewing gum.

Strength of taste of each chewing gum obtained was evaluated by 30 panelists (15 men, 15 women).

Taste was evaluated in 10 ranks of from 10 to 1 from the order of stronger taste every minute from the beginning of chewing, and an average value of 30 panelists was calculated. The results are shown in Table 1.

**Table 1**

| Gum | Menthol Composition | After 1 Min. | After 2 Min. | After 3 Min. | After 4 Min. | After 5 Min. |
|---|---|---|---|---|---|---|
| Ex. 1-A1 | Ex. 1-1 | 8.3 | 8.0 | 7.5 | 7.3 | 6.6 |
| Ex. 1-A2 | Ex. 1-2 | 8.3 | 7.6 | 7.3 | 6.9 | 6.3 |
| Ex. 1-A3 | Ex. 1-3 | 8.0 | 7.7 | 7.0 | 6.8 | 5.9 |
| Ex. 1-A4 | Ex. 1-4 | 8.3 | 7.9 | 7.4 | 7.2 | 6.6 |
| Ex. 1-A5 | Ex. 1-5 | 8.0 | 7.8 | 7.1 | 6.9 | 6.1 |
| Ex. 1-A6 | Ex. 1-6 | 8.1 | 7.9 | 7.4 | 7.0 | 6.5 |
| Ex. 1-A7 | Ex. 1-7 | 8.4 | 8.4 | 8.3 | 8.0 | 7.6 |
| Ex. 1-A8 | Ex. 1-8 | 8.3 | 8.1 | 7.7 | 7.4 | 6.6 |
| Ex. 1-A9 | Ex. 1-9 | 8.4 | 8.4 | 8.3 | 8.0 | 7.6 |
| Ex. 1-A10 | Ex. 1-10 | 8.3 | 8.1 | 7.6 | 7.4 | 6.6 |
| Comp. Ex. 1-A1 | Comp. Ex. 1-1 | 9.5 | 8.3 | 5.5 | 4.8 | 2.8 |
| Comp. Ex. 1-A2 | Comp. Ex. 1-2 | 9.8 | 8.5 | 4.9 | 4.5 | 2.9 |

It can be seen from the above results that the chewing gums in which the menthol-supporting porous silicas obtained in Examples were used sustain taste even when continued chewing for a long period of time, as compared to those in which the menthol-supporting porous substances of Comparative Examples were used.

### Examples 1-B1 to 1-B10 and Comparative Examples 1-B1 and 1-B2

Two grams of the menthol-supporting porous silica obtained in each Example or the menthol-supporting porous substance obtained in each Comparative Example was mixed with 96 g of water and 2 g of a gelating agent preparation (SUNKARA #2122, manufactured by Taiyo Kagaku Co., Ltd.). The mixture was heated at 85°C for 5 minutes while stirring. The heated mixture was molded and then cooled to give 100 g of an adhesive preparation.

Sustainability of refreshing feel and cool feel of each of the adhesive preparations was evaluated by 30 panelists (15 men, 15 women).

A piece of each poultice cut into a square having a length of 3 cm and a width of 3 cm was adhered to the upper arm of every panelist. The refreshing feel and cool feel were rated in 10 ranks from 10 to 1 according to the order of stronger feel of these feels every 10 minutes after the adhesion, and an average value of 30 panelists was calculated. The results are shown in Table 2.

**Table 2**

| Adhesive Preparation | Menthol Composition | After 10 Min. | After 20 Min. | After 30 Min. | After 40 Min. |
|---|---|---|---|---|---|
| Ex. 1-B1 | Ex. 1-1 | 8.3 | 8.0 | 7.8 | 7.3 |
| Ex. 1-B2 | Ex. 1-2 | 8.2 | 7.9 | 7.9 | 7.1 |
| Ex. 1-B3 | Ex. 1-3 | 8.0 | 7.7 | 7.5 | 6.9 |
| Ex. 1-B4 | Ex. 1-4 | 8.3 | 7.9 | 7.4 | 7.2 |
| Ex. 1-B5 | Ex. 1-5 | 8.0 | 7.8 | 7.1 | 6.9 |
| Ex. 1-B6 | Ex. 1-6 | 7.9 | 7.6 | 7.9 | 7.2 |
| Ex. 1-B7 | Ex. 1-7 | 8.4 | 8.3 | 8.3 | 8.2 |
| Ex. 1-B8 | Ex. 1-8 | 8.3 | 8.1 | 7.9 | 7.3 |
| Ex. 1-B9 | Ex. 1-9 | 8.4 | 8.3 | 8.3 | 8.2 |
| Ex. 1-B10 | Ex. 1-10 | 8.3 | 8.1 | 7.9 | 7.3 |
| Comp. Ex. 1-B1 | Comp. Ex. 1 | 9.5 | 8.3 | 5.4 | 4.8 |
| Comp. Ex. 1-B2 | Comp. Ex. 2 | 9.8 | 8.5 | 5.0 | 4.5 |

It can be seen from the above results that the adhesive preparations using the menthol-supporting porous silica obtained in Examples sustain refreshing feel and cool feel for a long period of time after adhesion to the human body, as compared to those using the menthol-supporting porous substance obtained in Comparative Examples.

### Examples 1-C1 to 1-C10

The amount 10.0 g of a solid paraffin, 20.4 g of castor oil, 14.0 g of lanoline, 5.0 g of beeswax, 12.0 g of candelilla wax, 7.0 g of carnauba wax, 18.0 g of 2-ethylhexanoate cetyl and 12.0 g of isopropyl myristate were dissolved. Thereafter, 1 g of the menthol-supporting porous silica obtained in each Example was added to the solution while mixing, and the mixture was poured into a mold and cooled to give 99.4 g of a lip cream.

### Examples 1-D1 to 1-D10 and Comparative Examples 1-D1 and 1-D2

A β-carotene pigment was added to 5 g of the menthol-supporting porous silica obtained in each Example or the menthol-supporting porous substance obtained in each Comparative Example, and granulated into a powder to give 5 g of a red deodorant.

A vaporization loss percentage (%) of menthol after each deodorant was allowed to stand at room temperature for 1 week, 2 weeks, 1 month and 3 months respectively was calculated according to the following formula. The results are shown in Table 3.

Vaporization loss percentage (%) = (Weight of deodorant before measurement - Weight of deodorant after allowing to stand for given period of time) / Weight of deodorant before measurement x 100

**Table 3**

| Deodorant | Menthol Composition | After 1 Week | After 2 Weeks | After 1 Month | After 3 Months |
|---|---|---|---|---|---|
| Ex. 1-D1 | Ex. 1-1 | 1.2 | 1.8 | 3.1 | 5.1 |
| Ex.1-D2 | Ex. 1-2 | 1.5 | 2.3 | 3.5 | 5.9 |
| Ex. 1-D3 | Ex. 1-3 | 1.8 | 2.9 | 3.7 | 6.3 |
| Ex. 1-D4 | Ex. 1-4 | 1.6 | 2.2 | 3.0 | 5.0 |
| Ex. 1-D5 | Ex. 1-5 | 1.8 | 2.0 | 3.7 | 5.3 |
| Ex. 1-D6 | Ex. 1-6 | 1.9 | 1.5 | 3.4 | 5.2 |
| Ex. 1-D7 | Ex. 1-7 | 1.1 | 1.3 | 1.6 | 3.3 |
| Ex. 1-D8 | Ex. 1-8 | 1.2 | 1.7 | 2.9 | 5.0 |
| Ex. 1-D9 | Ex. 1-9 | 1.1 | 1.3 | 1.6 | 3.3 |
| Ex. 1-D10 | Ex. 1-10 | 1.1 | 1.6 | 2.5 | 4.8 |
| Comp. Ex. 1-D1 | Comp. Ex. 1 | 10.5 | 21.8 | 30.1 | 32.5 |
| Comp. Ex. 1-D2 | Comp. Ex. 2 | 11.3 | 23.3 | 29.9 | 33.1 |

It can be seen from the above results that the deodorants using the menthol-supporting porous silicas obtained in Examples have smaller vaporization of menthol and excellent sustained-release property, as compared to those using the menthol-supporting porous substances obtained in Comparative Examples.

### Preparation Example 2-1

A solution prepared by dissolving 14 g of natural L-menthol in 200 g of ethanol was added to 20 g of the porous silica F while mixing, and thereafter the mixture was stirred at 40°C for 30 minutes with a homomixer. Subsequently, the solvent was removed by concentration with a rotary evaporator to give 34 g of a volatile substance-supporting porous silica (water content: 2% by weight).

### Preparation Example 2-2

Thirty grams of natural L-menthol was mixed with 70 g of the porous silica F, and thereafter the mixture was placed in a tightly sealed vessel and allowed to stand at 40°C for 1 week under a reduced pressure. The sublimed menthol was supported to a substrate containing silicon dioxide to give 100 g of a volatile substance-supporting porous silica.

### Preparation Example 2-3

A solution prepared by dissolving 14 g of natural L-menthol in 200 g of ethanol was added to 20 g of a commercially available fine powder silicon dioxide (trade name: CARPLEX, manufactured by SHIONOGI & CO., LTD.) while mixing, and thereafter the mixture was stirred at 40°C for 30 minutes with a homomixer. Subsequently, the solvent was removed by concentration with a rotary evaporator to give 34 g of a volatile substance composition (water content: 2% by weight).

### Example 2-1

Using the volatile substance-supporting porous silica obtained in Preparation Example 2-1, 94 g of water, 2 g of ethanol and 2 g of a gelating agent preparation (SUNKARA #2122, manufactured by Taiyo Kagaku Co., Ltd.) were heated at 85°C for 5 minutes to dissolve. Thereafter 3 g of the menthol-supporting porous silica obtained in Preparation Example 2-1 was added thereto, and the mixture was stirred. The mixture was molded and the thereafter cooled to give 100 g of a cooling gel sheet.

### Example 2-2

One gram of a polyglycerol fatty acid ester (SUNSOFT AZ-18EG, manufactured by Taiyo Kagaku Co., Ltd., HLB = 18) was dissolved in 50 g of water, and 3 g of the menthol-supporting porous silica obtained in Preparation Example 2-1 was added to this solution to disperse, to give a dispersion of the menthol-supporting porous silica. Thereafter, 44 g of water, 2 g of ethanol and 0.1 g of a gelating agent preparation (SUNKARA #2122, manufactured by Taiyo Kagaku Co., Ltd.) was heated at 85°C for 5 minutes to dissolve. Thereafter, an entire volume of the dispersion of the menthol-supporting porous silica was added thereto while stirring. The mixture was molded and then cooled to give 100 g of a cooling gel sheet.

### Example 2-3

The same procedures as in Example 2-2 were carried out except that a sucrose fatty acid ester (RYOTO Sugar Ester, manufactured by Mitsubishi-Kagaku Foods Corporation, L-1695) was added in place of the polyglycerol fatty acid ester to give 100 g of a cooling gel sheet.

### Example 2-4

The same procedures as in Example 2-3 were carried out except that the polyglycerol fatty acid ester was used in an amount of 1 g to give 101 g of a cooling gel sheet.

### Comparative Example 2-1

The same procedures as in Example 2-1 were carried out except that the volatile substance composition obtained in Preparation Example 2-3 was used in place of the volatile substance-supporting porous silica obtained in Preparation Example 2-1 to give a cooling gel sheet.

### Comparative Example 2-2

The same procedures as in Example 2-1 were carried out except that the raw material fine powder silicon dioxide used in Preparation Example 2-3 was used in place of the volatile substance-supporting porous silica obtained in Preparation Example 2-1 to give a cooling gel sheet.

Also, as a control, the same procedures as in Example 2-1 were carried out except that the volatile substance-supporting porous silica obtained in Preparation Example 2-1 was not used to give a cooling gel sheet.

### Test Example 2-1

Sustainability of refreshing feel and cool feel of each gel sheet was evaluated by 30 panelists (15 men, 15 women)

A piece of each gel sheet cut into a square having a length of 3 cm and a width of 3 cm was adhered to the upper arm of every panelist. The refreshing feel and cool feel were rated in 10 ranks from 10 to 1 according to the order of stronger feel of these feels every 5 minutes from after the adhesion to 20 minutes, and every 10 minutes from on and after 20 minutes to after 60 minutes, and an average value of 30 panelists was calculated. The results are shown in Table 4.

**Table 4**

| Composition Used | After 5 Min. | After 10 Min. | After 15 Min. | After 20 Min. | After 30 Min. | After 40 Min. | After 50 Min. | After 60 Min. |
|---|---|---|---|---|---|---|---|---|
| Ex. 2-1 | 9.7 | 9.8 | 9.8 | 9.7 | 9.7 | 9.1 | 8.8 | 8.5 |
| Ex. 2-2 | 9.9 | 9.9 | 9.9 | 9.9 | 9.9 | 9.7 | 9.6 | 9.5 |
| Ex. 2-3 | 9.7 | 9.8 | 9.8 | 9.7 | 9.7 | 9.1 | 8.9 | 8.6 |
| Ex. 2-4 | 9.7 | 9.8 | 9.8 | 9.7 | 9.7 | 9.1 | 8.9 | 8.5 |
| Comp. Ex. 2-1 | 9.5 | 8.6 | 6.2 | 4.1 | 4.1 | 3.8 | 3.2 | 2.9 |
| Comp. Ex. 2-2 | 8.3 | 7.8 | 7.1 | 6.5 | 6.5 | 5.1 | 4.9 | 3.3 |
| Control | 5.0 | 4.7 | 4.2 | 3.1 | 3.1 | 2.3 | 1.1 | 1.0 |

It was confirmed from the above results that the cooling gel sheets obtained in Examples sustained refreshing feel and cool feel for a long period of time after the adhesion to a human body, as compared to the cooling gel sheets obtained in Comparative Examples.

### Example 2-5. Preparation of Coolant (Icing Agent)

Twenty-two grams of white Vaseline, 18 g of stearyl alcohol, 3 g of polyoxyethylene hydrogenated castor oil and 1 g of glycerol monostearate were melted while heating in a water bath, and the melted mixture was stirred. Thereafter, 12 g of propylene glycol, 0.1 g of methyl parahydroxybenzoate and 0.1g of propyl parahydroxybenzoate were added thereto while stirring. To the mixture were added 2 g of the volatile substance-supporting porous silica obtained in Example 2-1, 2 g of methyl salicylate, 10 g of ethanol and 30 g of purified water, and the mixture was sufficiently stirred into a homogeneous state to give 100 g of an icing agent in ointment state. When this product was applied on the arm, the cool feel was sustained for one or more hours, giving a comfortable feel.

### Preparation Example 3-1

A solution prepared by dissolving 14 g of a natural capsicum extract in 200 g of ethanol was added to 20 g of the porous silica F while mixing, and thereafter the mixture was stirred at 40°C for 30 minutes with a homomixer. Subsequently, the solvent was removed by concentration with a rotary evaporator to give 34 g of a thermal substance-supporting porous silica (water content: 2% by weight).

### Preparation Example 3-2

One gram of a polyglycerol fatty acid ester (SUNSOFT AZ-18EG, manufactured by Taiyo Kagaku Co., Ltd.) was dissolved in 10 g of ethanol, and 20 g of the thermal substance-supporting porous silica obtained in Preparation Example 3-1 was added to this solution while mixing. Thereafter, the solvent was removed by concentration with a rotary evaporator to give 21 g of a thermal substance-supporting porous silica.

### Preparation Example 3-3

The same procedures as in Preparation Example 3-2 were carried out except that a sucrose fatty acid ester (RYOTO Sugar Ester, manufactured by Mitsubishi-Kagaku Foods Corporation, L-1695) was used in place of the polyglycerol fatty acid ester to give 21 g of a thermal substance-supporting porous silica.

### Preparation Example 3-4

A solution prepared by dissolving 14 g of a natural capsicum extract in 200 g of ethanol was added to 20 g of a commercially available fine powder silicon dioxide (trade name: CARPLEX, manufactured by SHIONOGI & CO., LTD.) while mixing, and thereafter the mixture was stirred at 40°C for 30 minutes with a homomixer. Subsequently, the solvent was removed by concentration with a rotary evaporator to give 34 g of a thermal substance-containing composition (water content: 2% by weight).

### Example 3-1

Ninety-four grams of water, 2 g of ethanol and 2 g of a gelating agent preparation (SUNKARA No. 2122, manufactured by Taiyo Kagaku Co., Ltd.) were heated at 85°C for 5 minutes to dissolve. Thereafter, 3 g of the thermal substance-supporting porous silica obtained in Preparation Example 3-1 was added thereto while stirring. The mixture was molded and then cooled to give 100 g of a thermal sheet.

### Example 3-2

The same procedures as in Example 3-1 were carried out except that the thermal substance-supporting porous silica obtained in Preparation Example 3-2 was used in place of the thermal substance-supporting porous silica obtained in Preparation Example 3-1 to give a thermal sheet.

### Example 3-3

The same procedures as in Example 3-1 were carried out except that the thermal substance-supporting porous silica obtained in Preparation Example 3-3 was used in place of the thermal substance-supporting porous silica obtained in Preparation Example 3-1 to give a thermal sheet.

### Example 3-4

The amount 0.15 g of a polyglycerol fatty acid ester (SUNSOFT AZ-18EG, manufactured by Taiyo Kagaku Co., Ltd., HLB = 18) was dissolved in 50 g of water, and 3 g of the thermal substance-supporting porous silica obtained in Preparation Example 3-1 was added to this solution to disperse to prepare a dispersion of the thermal substance-supporting porous silica. Thereafter, 44 g of water, 2 g of ethanol and 2 g of a gelating agent preparation (SUNKARA #2122, manufactured by Taiyo Kagaku Co., Ltd.) was heated at 85°C for 5 minutes to dissolve, and thereafter a whole volume of the dispersion of the thermal substance-supporting porous silica was added thereto while stirring. The mixture was molded and then cooled to give 100 g of a thermal sheet.

### Example 3-5

The same procedures as in Example 3-4 were carried out except that a sucrose fatty acid ester (RYOTO Sugar Ester, manufactured by Mitsubishi-Kagaku Foods Corporation, L-1695) was added in place of the polyglycerol fatty acid ester to give 100 g of a thermal sheet.

### Comparative Example 3-1

The same procedures were carried out except that the thermal substance-containing composition obtained in Preparation Example 3-4 was used in place of the thermal substance-supporting porous silica obtained in Preparation Example 3-1 to give a comparative product thermal sheet.

### Test Example 3-1

Sustainability of a thermal feel of each thermal sheet was evaluated by 30 panelists (15 men, 15 women).

A piece of each thermal sheet cut into a square having a length of 3 cm and a width of 3 cm was adhered to the upper arm of every panelist. The thermal feel was rated in 10 ranks from 10 to 1 according to the order of stronger feel of thermal feel every 5 minutes after the adhesion, and an average value of 30 panelists was calculated. The results are shown in Table 5. In addition, the presence or absence of skin irritation immediately after the adhesion was compared at the same time.

**Table 5**

| Sheet Used | Irritation | 1 Hour | 2 Hours | 3 Hours | 4 Hours | 5 Hours |
|---|---|---|---|---|---|---|
| Ex.3-1 | Absent | 8.3 | 8 | 8.1 | 7.8 | 8 |
| Ex. 3-2 | Absent | 8.2 | 8.2 | 8.2 | 8.2 | 8.2 |
| Ex. 3-3 | Absent | 8.3 | 8.2 | 8.2 | 8.1 | 8 |
| Ex. 3-4 | Absent | 8.2 | 8.2 | 8.2 | 8.2 | 8.2 |
| Ex. 3-5 | Absent | 8.3 | 8.2 | 8.2 | 8.1 | 8 |
| Comp. Ex. 3-1 | Present | 9.2 | 8.6 | 7.2 | 6.4 | 5.2 |

It was confirmed from the above results that the thermal sheets obtained in Examples gave no irritation and sustained a thermal feel for a long period of time after the adhesion to a human body as compared to the thermal sheet obtained in comparison.

### Example 3-6

Twenty-two grams of white Vaseline, 18 g of stearyl alcohol, 3 g of a polyoxyethylene hydrogenated castor oil and 1 g of glycerol monostearate were heated in a water bath to dissolve while stirring. Thereafter, 12 g of propylene glycol, 0.1 g of methyl parahydroxybenzoate and 0.1 g of propyl parahydroxybenzoate were added thereto while stirring. To the mixture obtained were added 2 g of the thermal substance-supporting porous silica obtained in Preparation Example 3-1, 2 g of methyl salicylate, 10 g of ethanol and 30 g of purified water, and sufficiently stirred into a homogeneous state to give 100 g of a thermal agent in ointment state. When this product was applied to the arm, the thermal feel was sustained, giving a comfortable feel.

### Preparation Example 4-1

Fifty grams of a vegetable polyphenol (green tea extract, SUNFLAVON HG, manufactured by Taiyo Kagaku Co., Ltd.) was dissolved in 50 g of water, 100 g of the porous silica F was added to the solution, and the mixture was stirred at 25°C for 30 minutes. Thereafter, the solvent was removed by concentration with a rotary evaporator to give 148 g of a vegetable polyphenol-supporting porous silica.

### Preparation Example 4-2

The amount 0.05 g of a polyglycerol fatty acid ester (SUNSOFT 818DG; manufactured by Taiyo Kagaku Co., Ltd.) was dissolved in 10 g of ethanol, and 1 g of the vegetable polyphenol-supporting porous silica obtained in Preparation Example 4-1 was added to this solution to disperse to prepare a dispersion of the vegetable polyphenol-supporting porous silica. To this dispersion were added 10 g of water and 0.05 g of a polyglycerol fatty acid ester (SUNSOFT AZ-18EG; manufactured by Taiyo Kagaku Co., Ltd.) while stirring. After stirring, the solvent was removed with a rotary evaporator to give 1.1 g of a vegetable polyphenol-supporting porous silica.

### Preparation Example 4-3

The same procedures as in Preparation Example 4-2 were carried out except that each polyglycerol fatty acid ester was used in an amount of 0.0005 g each to give 1 g of a vegetable polyphenol-supporting porous silica.

### Example 4-1

A dry nonwoven fabric having a basis weight of 45 g/m² which was made from polyester fibers and rayon fibers bound with acrylic resin as an air-permeable substrate was subjected to immersion-coating with a coating liquid prepared by mixing 85 g of the vegetable polyphenol-supporting porous silica obtained in Preparation Example 4-1 and 15 g of a binder containing an emulsion of styrene-acrylic copolymer resin while stirring, so as to contain the coating liquid in an amount of 30 g/m² on a dry basis, and dried to give a filter.

### Example 4-2

A dry nonwoven fabric having a basis weight of 45 g/m² which was made from polyester fibers and rayon fibers bound with acrylic resin as an air-permeable substrate was subjected to immersion-coating with a coating liquid prepared by mixing a liquid mixture of 15 g of a binder containing an emulsion of styrene-acrylic copolymer resin, 1.4 g of a polyglycerol fatty acid ester (SUNSOFT 818DG; manufactured by Taiyo Kagaku Co., Ltd.) and 1.4 g of a polyglycerol fatty acid ester (SUNSOFT AZ-18EG; manufactured by Taiyo Kagaku Co., Ltd.), and 85 g of the vegetable polyphenol-supporting porous silica obtained in Preparation Example 4-1 while stirring, so as to contain the coating liquid in an amount of 30 g/m² on a dry basis, and dried to give a filter.

### Comparative Example 4-1. Production of Filter

The same procedures as in Example 4-1 were carried out except that a commercially available zeolite was used in place of the vegetable polyphenol-supporting porous silica.

### Test Example 4-1 Adsorption Test of Ammonia and Acetaldehyde

A 5-liter teddler pack equipped with a small fan was charged with 3 liters of air thereinto, a sample prepared by cutting the filter obtained in Example 4-1 or Comparative Example 4-1 into a square of 10 cm x 10 cm was hung in this vessel, and thereafter ammonia and acetaldehyde were injected thereinto, respectively. The concentrations of both the components were determined with a gas detecting tube (No. 3L and 92M, manufactured by GASTEC CORPORATION). As a result, the concentration of ammonia was 60 ppm, and that of acetaldehyde was 70 ppm. Thereafter, the air in the vessel was forcibly circulated at 23°C by rotating the fan, and the concentrations (ppm) of ammonia and acetaldehyde in the vessel were determined at 10 minutes passed and at 30 minutes passed after the injection with the gas detecting tube described above. The results are shown in Table 6 together with the results of a control where the filter was not placed in the tightly sealed vessel.

**Table 6**

| Filter Used | Ammonia (ppm) | | Acetaldehyde (ppm) | |
|---|---|---|---|---|
| | After 10 Min. | After 30 Min. | After 10 Min. | After 30 Min. |
| Ex. 4-1 | 10 | 5 | 15 | 5 |
| Ex. 4-2 | 8 | 3 | 10 | 3 |
| Comp. Ex. 4-1 | 50 | 45 | 65 | 60 |
| Control | 60 | 60 | 70 | 70 |

It could be seen from the test results that the filters of the present invention had excellent adsorbability for ammonia, which was a causative of foul odor, and for acetaldehyde, which was a causative of sick house syndrome.

### Test Example 4-2

The filter obtained in Example 4-1 was kept at room temperature for 3 months in the vessel in a tightly sealed state after the above-mentioned test. The room temperature was adjusted to 23°C, and the vessel was allowed to stand at the temperature for 1 hour. Thereafter, ammonia and acetaldehyde were respectively injected into this vessel in the same manner as in Test Example 4-1. The concentrations (ppm) of ammonia and acetaldehyde in the vessel were determined immediately after the injection, at 10 minutes passed and at 30 minutes passed after the injection with a gas detecting tube. The results are shown in Table 7.

**Table 7**

| Filter Used | Ammonia (ppm) | | | Acetaldehyde (ppm) | | |
|---|---|---|---|---|---|---|
| | Immediately After | After 10 Min. | After 30 Min. | Immediately After | After 10 Min. | After 30 Min. |
| Ex. 4-1 | 60 | 10 | 5 | 70 | 15 | 5 |

It could be seen from the test results that the filter of the present invention sustained excellent adsorbability even after 3 months passed.

### Example 5-1

A dispersion prepared by dispersing 18 g of chlorophyll in 800 L of hexane was added to 100 g of the porous silica F while mixing, and thereafter the mixture was stirred at 25°C for 30 minutes with a homomixer. Subsequently, the solvent was removed by concentration with a rotary evaporator to give 118 g of a chlorophyll-supporting porous silica. One gram of pentaglycerol monomyristate (the ratio of polyglycerol having a degree of polymerization of 3 or more: 97%, the ratio of polyglycerol having a degree of polymerization of from 3 to 11: 94%), 1 g of an enzymatically decomposed lecithin and 88 g of water were added to 10 g of this chlorophyll-supporting porous silica to give 100 g of a pigment preparation A, of which average particle size was 430 nm.

### Examples 5-2 to 5-5

The same procedures as in Example 5-1 were carried out except that the porous silica B, C, D or E was used in place of the porous silica F, to give each of pigment preparations B to E, of which average particle sizes thereof were as follows: The pigment preparation B: 420 nm, the pigment preparation C: 380 nm, the pigment preparation D: 1.5 µm, and the pigment preparation E: 6.1 µm.

### Example 5-6

A dispersion prepared by dispersing 18 g of chlorophyll in 800 L of hexane was added to 100 g of the porous silica E while mixing, and thereafter the mixture was stirred at 25°C for 30 minutes with a homomixer. Subsequently, the solvent was removed by concentration with a rotary evaporator to give 118 g of a chlorophyll-supporting porous silica. Twelve grams of a polyglycerol fatty acid ester (SUNSOFT A-141E, manufactured by Taiyo Kagaku Co., Ltd., the ratio of polyglycerol having a degree of polymerization of 3 or more = 74%) and 78 g of water were added to 10 g of this chlorophyll-supporting porous silica, and the mixture was subjected to wet pulverization (with Ready Mill BSG-1/4, manufactured by Aimex Co., Ltd.) to give a pigment preparation, of which average particle size was 380 nm.

### Example 5-7

A solution prepared by dissolving 10 g of C. I. Acid Blue 9 in 1000 L of ion-exchanged water was added to 100 g of the porous silica G while mixing, and thereafter the mixture was stirred at 25°C for 30 minutes with a homomixer. Subsequently, the solvent was removed by concentration with a rotary evaporator to give 118 g of a C. I. Acid Blue 9-supporting porous silica. One gram of pentaglycerol monomyristate (the ratio of polyglycerol having a degree of polymerization of 3 or more: 97%, and the ratio of polyglycerol having a degree of polymerization of from 3 to 11: 94%), 1 g of an enzymatically decomposed lecithin and 88 g of water were added to 10 g of this porous silica to give 100 g of a pigment preparation, of which average particle size was 450 nm.

### Example 5-8

The same procedures as in Example 5-7 were carried out except that the porous silica F was used in place of the porous silica G to give 100 g of a pigment preparation, of which average particle size was 490 nm.

### Example 5-9

A solution prepared by dissolving 10 g of C.I. Acid Red 1 in 1000 L of ion-exchanged water was added to 100 g of the porous silica F while mixing, and thereafter the mixture was stirred at 25°C for 30 minutes with a homomixer. Subsequently, the solvent was removed by concentration with a rotary evaporator to give 115 g of a C. I. Acid Red 1-supporting porous silica. One gram of pentaglycerol monomyristate (the ratio of polyglycerol having a degree of polymerization of 3 or more: 97%, and the ratio of polyglycerol having a degree of polymerization of from 3 to 11: 94%), 1 g of an enzymatically decomposed lecithin and 88 g of water were added to 10 g of this porous silica to give 100 g of a pigment preparation, of which average particle size was 450 nm.

### Example 5-10

The same procedures as in Example 5-9 were carried out except that a polyglycerol condensed polyricinoleate (a polyglycerol fatty acid ester prepared by further esterifying a condensate prepared by esterifying a fatty acid having 18 or more carbon atoms with a polyglycerol, the polyglycerol containing 70% or more of a polyglycerol having a degree of polymerization of 3 or more,) was used in place of the pentaglycerol monomyristate to give 100 g of a pigment preparation, of which average particle size was 100 nm.

### Example 5-11

The same procedures as in Example 5-10 were carried out except that 30 g of the polyglycerol condensed polyricinoleate to give 110 g of a pigment preparation, of which average particle size was 95 nm.

### Example 5-12

The same procedures as in Example 5-9 were carried out except that a sucrose fatty acid ester (RYOTO Sugar Ester, manufactured by Mitsubishi-Kagaku Foods Corporation, L-1695) was used in place of the pentaglycerol monomyristate to give 100 g of a pigment preparation, of which average particle size was 600 nm.

### Example 5-13

A solution prepared by dissolving 10 g of C.I. Acid Red 1 in 1000 L of ion-exchanged water was added to 100 g of the porous silica F while mixing, and thereafter the mixture was stirred at 25°C for 30 minutes with a homomixer. Subsequently, the solvent was removed by concentration with a rotary evaporator to give 115 g of a C. I. Acid Red 1-supporting porous silica. The amount 0.1 g of pentaglycerol monomyristate (the ratio of polyglycerol having a degree of polymerization of 3 or more: 97%, and the ratio of polyglycerol having a degree of polymerization of from 3 to 11: 94%) and 99.9 g of water were added to 10 g of this porous silica to give 110 g of a pigment preparation, of which average particle size was 800 nm.

### Comparative Example 5-1

The same procedures as in Example 5-1 were carried out except that a sedimentary silica (CARPLEX CS-7, manufactured by SHIONOGI & CO., LTD.) was used in place of the porous silica F, to give a pigment preparation.

### Comparative Example 5-2

The same procedures as in Example 5-1 were carried out except that cyclodextrin (Dexy Pearl K-100, manufactured by ENSUIKO Sugar Refining Co., Ltd.) was used in place of the porous silica F, to give a pigment preparation.

### Comparative Example 5-3

The same procedures as in Example 5-7 were carried out except that a sedimentary silica (CARPLEX CS-7, manufactured by SHIONOGI & CO., LTD.) was used in place of the porous silica G, to give a pigment preparation.

### Comparative Example 5-4

The same procedures as in Example 5-9 were carried out except that a sedimentary silica (CARPLEX CS-7, manufactured by SHIONOGI & CO., LTD.) was used in place of the porous silica F, to give a pigment preparation.

### Example 5-14

Fifty grams of the pigment preparation obtained in each Example or each Comparative Example was added to 49.8 g of water, 0.1 g of an anticorrosive or mildewproof agent and 0.1 g of a pH adjusting agent, to prepare 100 g of an inkjet ink.

### Test Example 5-1

In order to evaluate dispersibility of each of the inkjet inks, the resulting ink in a state after being stored at 60°C for 1 month was evaluated by visual observation as follows:
- Ⓞ:: Generation of precipitates not being found at all
- ○:: Generation of precipitates being slightly found
- △:: Generation of precipitates being found
- ×:: Generation of precipitates being strikingly found

In addition, the resulting inkjet ink was loaded in a thermal inkjet test apparatus and jetted the ink from nozzles for printing. Here, the inkjet ink in which the pigment preparation obtained in Comparative Example 5-4 was added caused nozzle clogging in the course of printing.

In order to evaluate water resistance of each of the inkjet inks printed, the blurry state of the inks 60 seconds after dropping water droplets on a printout was evaluated by visual observation as follows:
- Ⓞ:: Blurriness of an ink not being found at all
- ○:: Blurriness of an ink being slightly observed
- Δ:: Blurriness of an ink being observed
- ×:: Blurriness of an ink being strikingly observed

The color development of the inkjet ink printed was evaluated by visual observation as follows:
- Ⓞ:: Very excellent color development being shown
- ○:: Excellent color development being shown
- △:: Color development being slightly worsened
- ×:: Color development being worsened

In order to evaluate light fastness of each of the inks, the resulting ink in a state after being stored for 1 month at 5000 lux was evaluated by visual observation as follows:
- Ⓞ:: Faded color not being found at all
- ○:: Faded color being slightly found
- △:: Faded color being found
- ×:: Faded color being strikingly found.

The results are shown in Tables 8 and 9.

**Table 8**

| Pigment Preparation | Dispersibility | Water Resistance | Color Development | Light Fastness |
|---|---|---|---|---|
| Ex. 5-7 | Ⓞ | Ⓞ | Ⓞ | Ⓞ |
| Ex. 5-8 | Ⓞ | Ⓞ | ○ | ○ |
| Comp. Ex. 5-3 | × | Δ | Δ | Δ |

**Table 9**

| Pigment Preparation | Dispersibility | Water Resistance | Color Development | Light Fastness |
|---|---|---|---|---|
| Ex. 5-9 | ○ | ○ | ○ | ○ |
| Ex. 5-10 | Ⓞ | ○ | Ⓞ | O |
| Ex. 5-11 | Ⓞ | Ⓞ | Ⓞ | Ⓞ |
| Ex. 5-12 | ○ | ○ | ○ | ○ |
| Ex. 5-13 | ○ | ○ | ○ | ○ |
| Comp. Ex. 5-4 | × | × | × | × |

It can be seen from the above results that the inkjet inks in which the water-dispersible preparations of Examples are used are excellent in water dispersibility, water resistance, color development and light fastness as compared to those in which the pigment preparations of Comparative Examples are used. The inkjet ink in which the porous silica bound and supported by 3-aminopropylmethyldimethoxysilane is used is even more excellent in color development and light fastness.

### Example 5-15

One gram of the pigment preparation obtained in each Example or each Comparative Example was added to 8.5 g of high fructose corn syrup, 0.6 g of a 50% citric acid solution, 0.04 g of sodium citrate, 0.1 g of ascorbic acid, 2.2 g of 1/5 melon fruit juice, 0.1 g of a melon flavor and 87.46 g of water while mixing, and the mixture was heated up to 93°C, and thereafter this solution was filled in a PET bottle and kept at 75°C for 5 minutes to prepare 100 g of a refreshing drink.

### Test Example 5-2

Dispersibility and light fastness of the refreshing drink obtained were evaluated.

### (Evaluation on Dispersibility)

- Ⓞ:: Generation of precipitates not being found at all
- ○:: Generation of precipitates being slightly found
- △:: Generation of precipitates being found
- ×:: Generation of precipitates being strikingly found

### (Evaluation on Light Fastness)

- Ⓞ:: Faded color not being found at all
- ○:: Faded color being slightly found
- △:: Faded color being found
- ×:: Faded color being strikingly found

The results are shown in Table 10.

**Table 10**

| Pigment Preparation | Dispersibility | Light Fastness |
|---|---|---|
| Ex. 5-1 | Ⓞ | Ⓞ |
| Ex. 5-2 | Ⓞ | Ⓞ |
| Ex. 5-3 | Ⓞ | Ⓞ |
| Ex. 5-4 | ○ | Ⓞ |
| Ex. 5-5 | ○ | Ⓞ |
| Ex. 5-6 | Ⓞ | Ⓞ |
| Comp. Ex. 5-1 | × | ○ |
| Comp. Ex. 5-2 | Δ | Δ |
| Comp. Ex. 5-3 | × | Δ |

It can be seen from the above results that the refreshing drinks in which the pigment preparations of Examples are used are excellent in water dispersibility, water resistance and light fastness, as compared to the drinks in which the pigment preparations of Comparative Examples are used.

### INDUSTRIAL APPLICABILITY

The substance-supporting porous silica of the present invention is excellent in sustained-release property of substances supported thereto, so that the porous silica is suitably used for various kinds of manufactured articles such as foodstuffs, medicaments, cosmetics, luxury items, toiletry articles and inks.

## Claims

1. A substance-supporting porous silica, wherein a porous silica supports a substance selected from the group consisting of menthols, volatile substances, thermal substances, plant polyphenols and organic colorants.

2. The substance-supporting porous silica according to claim 1, further comprising an emulsifying agent.

3. The substance-supporting porous silica according to claim 1 or 2, wherein the porous silica has a pore having an average pore size of from 0.8 to 20 nm.

4. The substance-supporting porous silica according to any one of claims 1 to 3, wherein the porous silica has an average particle size of from 50 nm to 100 µm.

5. The substance-supporting porous silica according to any one of claims 1 to 4, wherein the pore of the porous silica forms a hexagonal structure.

6. A composition comprising the substance-supporting porous silica as defined in any one of claims 1 to 5.

7. A coolant comprising a porous silica.
